# EUROPEAN PATENT APPLICATION

(11) **EP 1 043 396 A1**
(43) Date of publication of application: **11.10.2000**
(21) Application number: 99951121.5
(22) Date of filing: 28.10.1999
(51) Int. Cl.: C12N 15/12, C07K 14/47, C07K 16/18

(54) **GENES EXPRESSED IN HUMAN MONOCYTES AND HUMAN MACROPHAGES**

(30) Priority: 28.10.1998 JP 30753298
(71) Applicant: Japan Science and Technology Corporation, Kawaguchi-shi, Saitama 332-0012 (JP)
(72) Inventor: HASHIMOTO, Shinichi, Taito-ku, Tokyo 110-0001 (JP); MATSUSHIMA, Koji, Matsudo-shi, Chiba 271-0092 (JP); SUZUKI, Takuji, Ohta-ku, Tokyo 143-0025 (JP)
(74) Representative: Gardner, Rebecca
(86) International application number: JP9905982
(87) International publication number: WO0024892

(57) **Abstract**

The invention of the application provides gene groups individually comprising 100 genes expressed most in human monocytes, GM-macrophages and M-macrophages, respectively. The cDNAs of the individual genes belonging to these gene groups contain nucleotide sequences of SQ ID Nos. 1 to 100, 101 to 200, and 201 to 300 consecutive to the nucleotide sequence CATG closest to the polyA region. Additionally, the invention of the application provides the cDNA groups of the genes belonging to the gene groups, the novel gene groups contained in these gene groups, and the oligonucleotide groups identifying these genes and cDNAs.

Sequence Listing
<110> Japan Science and Technology Corporation
<120> Groups of genes expressed in human monocytes and human macrophages
<130> 99-F-035PCT/YS
<150> JP No. 10-307532
<151> 1998-10-28
<160> 480
<210> 1
<211> 10
<212> DNA
<213> Homo sapiens
<400> 1
GTGGCCACGG
<210> 2
<211> 10
<212> DNA
<213> Homo sapiens
<400> 2
TTGGGGTTTC
<210> 3
<211> 10
<212> DNA
<213> Homo sapiens
<400> 3
TGTGTTGAGA
<210> 4
<211> 10
<212> DNA
<213> Homo sapiens
<400> 4

TTGGTCCTCT
<210> 5
<211> 10
<212> DNA
<213> Homo sapiens
<400> 5
CCCTGGGTTC
<210> 6
<211> 10
<212> DNA
<213> Homo sapiens
<400> 6
CCCGTCCGGA
<210> 7
<211> 10
<212> DNA
<213> Homo sapiens
<400> 7
CACAAACGGT
<210> 8
<211> 10
<212> DNA
<213> Homo sapiens
<400> 8
CCTGTAATCC
<210> 9
<211> 10
<212> DNA
<213> Homo sapiens
<400> 9

GTTCACATTA
<210> 10
<211> 10
<212> DNA
<213> Homo sapiens
<400> 10
CTGACCTGTG
<210> 11
<211> 10
<212> DNA
<213> Homo sapiens
<400> 11
GTGAAACCCC
<210> 12
<211> 10
<212> DNA
<213> Homo sapiens
<400> 12
TGGTGTTGAG
<210> 13
<211> 10
<212> DNA
<213> Homo sapiens
<400> 13
TGCACGTTTT
<210> 14
<211> 10
<212> DNA
<213> Homo sapiens
<400> 14

TACCTGCAGA
<210> 15
<211> 10
<212> DNA
<213> Homo sapiens
<400> 15
CCCATCGTCC
<210> 16
<211> 10
<212> DNA
<213> Homo sapiens
<400> 16
GTTGTGGTTA
<210> 17
<211> 10
<212> DNA
<213> Homo sapiens
<400> 17
GTGAAGGCAG
<210> 18
<211> 10
<212> DNA
<213> Homo sapiens
<400> 18
GGGCATCTCT
<210> 19
<211> 10
<212> DNA
<213> Homo sapiens
<400> 19

TTGTAATCGT
<210> 20
<211> 10
<212> DNA
<213> Homo sapiens
<400> 20
AGGCTACGGA
<210> 21
<211> 10
<212> DNA
<213> Homo sapiens
<400> 21
CCCACAACCT
<210> 22
<211> 10
<212> DNA
<213> Homo sapiens
<400> 22
CCACTGCACT
<210> 23
<211> 10
<212> DNA
<213> Homo sapiens
<400> 23
GGGCTGGGGT
<210> 24
<211> 10
<212> DNA
<213> Homo sapiens
<400> 24

ATGGCTGGTA
<210> 25
<211> 10
<212> DNA
<213> Homo sapiens
<400> 25
CGCCGCCGGC
<210> 26
<211> 10
<212> DNA
<213> Homo sapiens
<400> 26
CCAGAACAGA
<210> 27
<211> 10
<212> DNA
<213> Homo sapiens
<400> 27
GAGGGAGTTT
<210> 28
<211> 10
<212> DNA
<213> Homo sapiens
<400> 28
AGGGCTTCCA
<210> 29
<211> 10
<212> DNA
<213> Homo sapiens
<400> 29

ACTTTTTCAA
<210> 30
<211> 10
<212> DNA
<213> Homo sapiens
<400> 30
TTGGTGAAGG
<210> 31
<211> 10
<212> DNA
<213> Homo sapiens
<400> 31
GTGCACTGAG
<210> 32
<211> 10
<212> DNA
<213> Homo sapiens
<400> 32
AGCACCTCCA
<210> 33
<211> 10
<212> DNA
<213> Homo sapiens
<400> 33
TAGGTTGTCT
<210> 34
<211> 10
<212> DNA
<213> Homo sapiens
<400> 34

GGGGAAATCG
<210> 35
<211> 10
<212> DNA
<213> Homo sapiens
<400> 35
TGCCTGCACC
<210> 36
<211> 10
<212> DNA
<213> Homo sapiens
<400> 36
   ATGTAAAAAA
<210> 37
<211> 10
<212> DNA
<213> Homo sapiens
<400> 37
GGATTTGGCC
<210> 38
<211> 10
<212> DNA
<213> Homo sapiens
<400> 38
CTGGGTTAAT
<210> 39
<211> 10
<212> DNA
<213> Homo sapiens
<400> 39

ACCGCCGTGG
<210> 40
<211> 10
<212> DNA
<213> Homo sapiens
<400> 40
GTGCGCTGAG
<210> 41
<211> 10
<212> DNA
<213> Homo sapiens
<400> 41
AAGACAGTGG
<210> 42
<211> 10
<212> DNA
<213> Homo sapiens
<400> 42
GGCAAGCCCC
<210> 43
<211> 10
<212> DNA
<213> Homo sapiens
<400> 43
TGGGCAAAGC
<210> 44
<211> 10
<212> DNA
<213> Homo sapiens
<400> 44

CCCCCTGGAT
<210> 45
<211> 10
<212> DNA
<213> Homo sapiens
<400> 45
TACCATCAAT
<210> 46
<211> 10
<212> DNA
<213> Homo sapiens
<400> 46
GCGGTTGTGG
<210> 47
<211> 10
<212> DNA
<213> Homo sapiens
<400> 47
GCCGTGTCCG
<210> 48
<211> 10
<212> DNA
<213> Homo sapiens
<400> 48
GTGAAACCCT
<210> 49
<211> 10
<212> DNA
<213> Homo sapiens
<400> 49

GAACACATCC
<210> 50
<211> 10
<212> DNA
<213> Homo sapiens
<400> 50
GCAGCCATCC
<210> 51
<211> 10
<212> DNA
<213> Homo sapiens
<400> 51
GTGTTAACCA
<210> 52
<211> 10
<212> DNA
<213> Homo sapiens
<400> 52
CCGTCCAAGG
<210> 53
<211> 10
<212> DNA
<213> Homo sapiens
<400> 53
CCCATCCGAA
<210> 54
<211> 10
<212> DNA
<213> Homo sapiens
<400> 54

TCAGATCTTT
<210> 55
<211> 10
<212> DNA
<213> Homo sapiens
<400> 55
AGGAAAGCTG
<210> 56
<211> 10
<212> DNA
<213> Homo sapiens
<400> 56
GCATAATAGG
<210> 57
<211> 10
<212> DNA
<213> Homo sapiens
<400> 57
CGCTGGTTCC
<210> 58
<211> 10
<212> DNA
<213> Homo sapiens
<400> 58
GTTCCCTGGC
<210> 59
<211> 10
<212> DNA
<213> Homo sapiens
<400> 59

AGCTCTCCCT
<210> 60
<211> 10
<212> DNA
<213> Homo sapiens
<400> 60
GCCTGTATGA
<210> 61
<211> 10
<212> DNA
<213> Homo sapiens
<400> 61
ATTTGAGAAG
<210> 62
<211> 10
<212> DNA
<213> Homo sapiens
<400> 62
ATTCTCCAGT
<210> 63
<211> 10
<212> DNA
<213> Homo sapiens
<400> 63
AAGGAGATGG
<210> 64
<211> 10
<212> DNA
<213> Homo sapiens
<400> 64

ATCAAGAATC
<210> 65
<211> 10
<212> DNA
<213> Homo sapiens
<400> 65
GTGCTGAATG
<210> 66
<211> 10
<212> DNA
<213> Homo sapiens
<400> 66
CTGTTGGTGA
<210> 67
<211> 10
<212> DNA
<213> Homo sapiens
<400> 67
ATCAAGGGTG
<210> 68
<211> 10
<212> DNA
<213> Homo sapiens
<400> 68
AAGGTGGAGG
<210> 69
<211> 10
<212> DNA
<213> Homo sapiens
<400> 69

ATGTGTAACG
<210> 70
<211> 10
<212> DNA
<213> Homo sapiens
<400> 70
CTAAGACTTC
<210> 71
<211> 10
<212> DNA
<213> Homo sapiens
<400> 71
CTCCTCACCT
<210> 72
<211> 10
<212> DNA
<213> Homo sapiens
<400> 72
TTCAATAAAA
<210> 73
<211> 10
<212> DNA
<213> Homo sapiens
<400> 73
GGACCACTGA
<210> 74
<211> 10
<212> DNA
<213> Homo sapiens
<400> 74

CTGTTGGCAT
<210> 75
<211> 10
<212> DNA
<213> Homo sapiens
<400> 75
CAATAAATGT
<210> 76
<211> 10
<212> DNA
<213> Homo sapiens
<400> 76
ACATCATCGA
<210> 77
<211> 10
<212> DNA
<213> Homo sapiens
<400> 77
CCAGTGGCCC
<210> 78
<211> 10
<212> DNA
<213> Homo sapiens
<400> 78
TGTGCTAAAT
<210> 79
<211> 10
<212> DNA
<213> Homo sapiens
<400> 79

AAGCACAAAA
<210> 80
<211> 10
<212> DNA
<213> Homo sapiens
<400> 80
GCACCAAAGC
<210> 81
<211> 10
<212> DNA
<213> Homo sapiens
<400> 81
CGCCGGAACA
<210> 82
<211> 10
<212> DNA
<213> Homo sapiens
<400> 82
TTACCATATC
<210> 83
<211> 10
<212> DNA
<213> Homo sapiens
<400> 83
ACGCAGGGAG
<210> 84
<211> 10
<212> DNA
<213> Homo sapiens
<400> 84

TCACCCACAC
<210> 85
<211> 10
<212> DNA
<213> Homo sapiens
<400> 85
TGATTTCACT
<210> 86
<211> 10
<212> DNA
<213> Homo sapiens
<400> 86
GCCCCCAATA
<210> 87
<211> 10
<212> DNA
<213> Homo sapiens
<400> 87
ACCCCCCCGC
<210> 88
<211> 10
<212> DNA
<213> Homo sapiens
<400> 88
TGGAAGCACT
<210> 89
<211> 10
<212> DNA
<213> Homo sapiens
<400> 89

TGCAGCACGA
<210> 90
<211> 10
<212> DNA
<213> Homo sapiens
<400> 90
GCCGAGGAAG
<210> 91
<211> 10
<212> DNA
<213> Homo sapiens
<400> 91
TCAGACGCAG
<210> 92
<211> 10
<212> DNA
<213> Homo sapiens
<400> 92
CCCCAGCCAG
<210> 93
<211> 10
<212> DNA
<213> Homo sapiens
<400> 93
CCAAACGTGT
<210> 94
<211> 10
<212> DNA
<213> Homo sapiens
<400> 94

GACGACACGA
<210> 95
<211> 10
<212> DNA
<213> Homo sapiens
<400> 95
GTTCGTGCCA
<210> 96
<211> 10
<212> DNA
<213> Homo sapiens
<400> 96
AAGTTGCTAT
<210> 97
<211> 10
<212> DNA
<213> Homo sapiens
<400> 97
TCACAGCTGT
<210> 98
<211> 10
<212> DNA
<213> Homo sapiens
<400> 98
CGACGAGGAG
<210> 99
<211> 10
<212> DNA
<213> Homo sapiens
<400> 99

CTCAACATCT
<210> 100
<211> 10
<212> DNA
<213> Homo sapiens
<400> 100
AGCCCTACAA
<210> 101
<211> 10
<212> DNA
<213> Homo sapiens
<400> 101
CCCTGGGTTC
<210> 102
<211> 10
<212> DNA
<213> Homo sapiens
<400> 102
TGGCCCCAGG
<210> 103
<211> 10
<212> DNA
<213> Homo sapiens
<400> 103
TTGGGGTTTC
<210> 104
<211> 10
<212> DNA
<213> Homo sapiens
<400> 104

CGACCCCACG
<210> 105
<211> 10
<212> DNA
<213> Homo sapiens
<400> 105
CCCATCGTCC
<210> 106
<211> 10
<212> DNA
<213> Homo sapiens
<400> 106
CTAAGACTTC
<210> 107
<211> 10
<212> DNA
<213> Homo sapiens
<400> 107
TGGGTGAGCC
<210> 108
<211> 10
<212> DNA
<213> Homo sapiens
<400> 108
ACTTTTTCAA
<210> 109
<211> 10
<212> DNA
<213> Homo sapiens
<400> 109

GGGGCAACAG
<210> 110
<211> 10
<212> DNA
<213> Homo sapiens
<400> 110
GCGGTTGTGG
<210> 111
<211> 10
<212> DNA
<213> Homo sapiens
<400> 111
GTTCACATTA
<210> 112
<211> 10
<212> DNA
<213> Homo sapiens
<400> 112
GTATGGGCCC
<210> 113
<211> 10
<212> DNA
<213> Homo sapiens
<400> 113
TTGGTCCTCT
<210> 114
<211> 10
<212> DNA
<213> Homo sapiens
<400> 114

GTTGTGGTTA
<210> 115
<211> 10
<212> DNA
<213> Homo sapiens
<400> 115
CCTGTAATCC
<210> 116
<211> 10
<212> DNA
<213> Homo sapiens
<400> 116
GGGGAAATCG
<210> 117
<211> 10
<212> DNA
<213> Homo sapiens
<400> 117
GAAATACAGT
<210> 118
<211> 10
<212> DNA
<213> Homo sapiens
<400> 118
TTCATACACC
<210> 119
<211> 10
<212> DNA
<213> Homo sapiens
<400> 119

TGTGTTGAGA
<210> 120
<211> 10
<212> DNA
<213> Homo sapiens
<400> 120
CTGACCTGTG
<210> 121
<211> 10
<212> DNA
<213> Homo sapiens
<400> 121
CTGGGCCTGG
<210> 122
<211> 10
<212> DNA
<213> Homo sapiens
<400> 122
CACCTAATTG
<210> 123
<211> 10
<212> DNA
<213> Homo sapiens
<400> 123
ACCGCCGTGG
<210> 124
<211> 10
<212> DNA
<213> Homo sapiens
<400> 124

CTTCCAGCTA
<210> 125
<211> 10
<212> DNA
<213> Homo sapiens
<400> 125
GTGCTGAATG
<210> 126
<211> 10
<212> DNA
<213> Homo sapiens
<400> 126
TTGTAATCGT
<210> 127
<211> 10
<212> DNA
<213> Homo sapiens
<400> 127
GTGAAACCCC
<210> 128
<211> 10
<212> DNA
<213> Homo sapiens
<400> 128
AGCCCTACAA
<210> 129
<211> 10
<212> DNA
<213> Homo sapiens
<400> 129

GCCCCCAATA
<210> 130
<211> 10
<212> DNA
<213> Homo sapiens
<400> 130
CTCCCCTGCC
<210> 131
<211> 10
<212> DNA
<213> Homo sapiens
<400> 131
CAAGCATCCC
<210> 132
<211> 10
<212> DNA
<213> Homo sapiens
<400> 132
GCGACCGTCA
<210> 133
<211> 10
<212> DNA
<213> Homo sapiens
<400> 133
GTGCGCTGAG
<210> 134
<211> 10
<212> DNA
<213> Homo sapiens
<400> 134

TAAATCCCCA
<210> 135
<211> 10
<212> DNA
<213> Homo sapiens
<400> 135
GTGACCACGG
<210> 136
<211> 10
<212> DNA
<213> Homo sapiens
<400> 136
TTCACTGTGA
<210> 137
<211> 10
<212> DNA
<213> Homo sapiens
<400> 137
GTGCACTGAG
<210> 138
<211> 10
<212> DNA
<213> Homo sapiens
<400> 138
TTGGTGAAGG
<210> 139
<211> 10
<212> DNA
<213> Homo sapiens
<400> 139

CACAAACGGT
<210> 140
<211> 10
<212> DNA
<213> Homo sapiens
<400> 140
CTGGCTGCAA
<210> 141
<211> 10
<212> DNA
<213> Homo sapiens
<400> 141
TGATTTCACT
<210> 142
<211> 10
<212> DNA
<213> Homo sapiens
<400> 142
CCTCAGGATA
<210> 143
<211> 10
<212> DNA
<213> Homo sapiens
<400> 143
TGGCCCCACC
<210> 144
<211> 10
<212> DNA
<213> Homo sapiens
<400> 144
   AACGGGGCCC
<210> 145
<211> 10
<212> DNA
<213> Homo sapiens
<400> 145
ACTATTTCCA
<210> 146
<211> 10
<212> DNA
<213> Homo sapiens
<400> 146
TGGCTCCTCC
<210> 147
<211> 10
<212> DNA
<213> Homo sapiens
<400> 147
GAAGCAGGAC
<210> 148
<211> 10
<212> DNA
<213> Homo sapiens
<400> 148
CGCCGCCGGC
<210> 149
<211> 10
<212> DNA
<213> Homo sapiens
<400> 149

GGAGGTGGGG
<210> 150
<211> 10
<212> DNA
<213> Homo sapiens
<400> 150
TCAGTTTGGA
<210> 151
<211> 10
<212> DNA
<213> Homo sapiens
<400> 151
ATGAGCTGAC
<210> 152
<211> 10
<212> DNA
<213> Homo sapiens
<400> 152
CCACTGCACT
<210> 153
<211> 10
<212> DNA
<213> Homo sapiens
<400> 153
CCCGTCCGGA
<210> 154
<211> 10
<212> DNA
<213> Homo sapiens
<400> 154

GGGCATCTCT
<210> 155
<211> 10
<212> DNA
<213> Homo sapiens
<400> 155
AAGTTGCTAT
<210> 156
<211> 10
<212> DNA
<213> Homo sapiens
<400> 156
GCCTGCTGGG
<210> 157
<211> 10
<212> DNA
<213> Homo sapiens
<400> 157
CCTAGCTGGA
<210> 158
<211> 10
<212> DNA
<213> Homo sapiens
<400> 158
GTGCGCTAGG
<210> 159
<211> 10
<212> DNA
<213> Homo sapiens
<400> 159

TACCATCAAT
<210> 160
<211> 10
<212> DNA
<213> Homo sapiens
<400> 160
GGGCTGGGGT
<210> 161
<211> 10
<212> DNA
<213> Homo sapiens
<400> 161
AGCACCTCCA
<210> 162
<211> 10
<212> DNA
<213> Homo sapiens
<400> 162
ACCCTTGGCC
<210> 163
<211> 10
<212> DNA
<213> Homo sapiens
<400> 163
GGCTGGGGGC
<210> 164
<211> 10
<212> DNA
<213> Homo sapiens
<400> 164

GCCATCCAGA
<210> 165
<211> 10
<212> DNA
<213> Homo sapiens
<400> 165
ATTTGAGAAG
<210> 166
<211> 10
<212> DNA
<213> Homo sapiens
<400> 166
TTTGGGGCTG
<210> 167
<211> 10
<212> DNA
<213> Homo sapiens
<400> 167
GCTTTATTTG
<210> 168
<211> 10
<212> DNA
<213> Homo sapiens
<400> 168
TAGGTTGTCT
<210> 169
<211> 10
<212> DNA
<213> Homo sapiens
<400> 169

TGCCTGCACC
<210> 170
<211> 10
<212> DNA
<213> Homo sapiens
<400> 170
AAGGACCTTT
<210> 171
<211> 10
<212> DNA
<213> Homo sapiens
<400> 171
TGCACGTTTT
<210> 172
<211> 10
<212> DNA
<213> Homo sapiens
<400> 172
AGTTTCTTGT
<210> 173
<211> 10
<212> DNA
<213> Homo sapiens
<400> 173
GTGAAACCCT
<210> 174
<211> 10
<212> DNA
<213> Homo sapiens
<400> 174

CCTTGTCCTC
<210> 175
<211> 10
<212> DNA
<213> Homo sapiens
<400> 175
TGCAGGCCTG
<210> 176
<211> 10
<212> DNA
<213> Homo sapiens
<400> 176
ATGGCTGGTA
<210> 177
<211> 10
<212> DNA
<213> Homo sapiens
<400> 177
CTCTTCGAGA
<210> 178
<211> 10
<212> DNA
<213> Homo sapiens
<400> 178
TCCTGGGTTC
<210> 179
<211> 10
<212> DNA
<213> Homo sapiens
<400> 179

AAAACATTCT
<210> 180
<211> 10
<212> DNA
<213> Homo sapiens
<400> 180
TGGTGTTGAG
<210> 181
<211> 10
<212> DNA
<213> Homo sapiens
<400> 181
CCGTCCAAGG
<210> 182
<211> 10
<212> DNA
<213> Homo sapiens
<400> 182
AGAAGTGTCC
<210> 183
<211> 10
<212> DNA
<213> Homo sapiens
<400> 183
AGGGCTTCCA
<210> 184
<211> 10
<212> DNA
<213> Homo sapiens
<400> 184

CTGGGTTAAT
<210> 185
<211> 10
<212> DNA
<213> Homo sapiens
<400> 185
GTGAAGGCAG
<210> 186
<211> 10
<212> DNA
<213> Homo sapiens
<400> 186
GGATTTGGCC
<210> 187
<211> 10
<212> DNA
<213> Homo sapiens
<400> 187
AGGCTACGGA
<210> 188
<211> 10
<212> DNA
<213> Homo sapiens
<400> 188
GCCTGTATGA
<210> 189
<211> 10
<212> DNA
<213> Homo sapiens
<400> 189

TCACCGGTCA
<210> 190
<211> 10
<212> DNA
<213> Homo sapiens
<400> 190
GCCCCAGCCC
<210> 191
<211> 10
<212> DNA
<213> Homo sapiens
<400> 191
GAGGGAGTTT
<210> 192
<211> 10
<212> DNA
<213> Homo sapiens
<400> 192
ATCAAGAATC
<210> 193
<211> 10
<212> DNA
<213> Homo sapiens
<400> 193
CTGCCAAGTT
<210> 194
<211> 10
<212> DNA
<213> Homo sapiens
<400> 194

AAGCACAAAA
<210> 195
<211> 10
<212> DNA
<213> Homo sapiens
<400> 195
CCCCCTGGAT
<210> 196
<211> 10
<212> DNA
<213> Homo sapiens
<400> 196
CGCTGGTTCC
<210> 197
<211> 10
<212> DNA
<213> Homo sapiens
<400> 197
TCACCCACAC
<210> 198
<211> 10
<212> DNA
<213> Homo sapiens
<400> 198
AAGACAGTGG
<210> 199
<211> 10
<212> DNA
<213> Homo sapiens
<400> 199

GCAGCCATCC
<210> 200
<211> 10
<212> DNA
<213> Homo sapiens
<400> 200
TGTCCCAGCC
<210> 201
<211> 10
<212> DNA
<213> Homo sapiens
<400> 201
TGGCCCCAGG
<210> 202
<211> 10
<212> DNA
<213> Homo sapiens
<400> 202
TTGGGGTTTC
<210> 203
<211> 10
<212> DNA
<213> Homo sapiens
<400> 203
CCCTGGGTTC
<210> 204
<211> 10
<212> DNA
<213> Homo sapiens
<400> 204

CGACCCCACG
<210> 205
<211> 10
<212> DNA
<213> Homo sapiens
<400> 205
TGGGTGAGCC
<210> 206
<211> 10
<212> DNA
<213> Homo sapiens
<400> 206
CAAGCATCCC
<210> 207
<211> 10
<212> DNA
<213> Homo sapiens
<400> 207
CCCATCGTCC
<210> 208
<211> 10
<212> DNA
<213> Homo sapiens
<400> 208
CCTGTAATCC
<210> 209
<211> 10
<212> DNA
<213> Homo sapiens
<400> 209

CTAAGACTTC
<210> 210
<211> 10
<212> DNA
<213> Homo sapiens
<400> 210
TTGGTCCTCT
<210> 211
<211> 10
<212> DNA
<213> Homo sapiens
<400> 211
TGTGTTGAGA
<210> 212
<211> 10
<212> DNA
<213> Homo sapiens
<400> 212
AGCCCTACAA
<210> 213
<211> 10
<212> DNA
<213> Homo sapiens
<400> 213
GGGGAAATCG
<210> 214
<211> 10
<212> DNA
<213> Homo sapiens
<400> 214

ACTTTTTCAA
<210> 215
<211> 10
<212> DNA
<213> Homo sapiens
<400> 215
TTCATACACC
<210> 216
<211> 10
<212> DNA
<213> Homo sapiens
<400> 216
CACCTAATTG
<210> 217
<211> 10
<212> DNA
<213> Homo sapiens
<400> 217
GCCCCCAATA
<210> 218
<211> 10
<212> DNA
<213> Homo sapiens
<400> 218
GTGAAACCCC
<210> 219
<211> 10
<212> DNA
<213> Homo sapiens
<400> 219

GTTGTGGTTA
<210> 220
<211> 10
<212> DNA
<213> Homo sapiens
<400> 220
TTGTAATCGT
<210> 221
<211> 10
<212> DNA
<213> Homo sapiens
<400> 221
CTCCCCTGCC
<210> 222
<211> 10
<212> DNA
<213> Homo sapiens
<400> 222
TTCACTGTGA
<210> 223
<211> 10
<212> DNA
<213> Homo sapiens
<400> 223
GCGGTTGTGG
<210> 224
<211> 10
<212> DNA
<213> Homo sapiens
<400> 224

GAAATACAGT
<210> 225
<211> 10
<212> DNA
<213> Homo sapiens
<400> 225
GTATGGGCCC
<210> 226
<211> 10
<212> DNA
<213> Homo sapiens
<400> 226
CCTAGCTGGA
<210> 227
<211> 10
<212> DNA
<213> Homo sapiens
<400> 227
TTGGTGAAGG
<210> 228
<211> 10
<212> DNA
<213> Homo sapiens
<400> 228
CCACTGCACT
<210> 229
<211> 10
<212> DNA
<213> Homo sapiens
<400> 229

GGCTGGGGGC
<210> 230
<211> 10
<212> DNA
<213> Homo sapiens
<400> 230
ATGAGCTGAC
<210> 231
<211> 10
<212> DNA
<213> Homo sapiens
<400> 231
ATCAAGAATC
<210> 232
<211> 10
<212> DNA
<213> Homo sapiens
<400> 232
TGATTTCACT
<210> 233
<211> 10
<212> DNA
<213> Homo sapiens
<400> 233
TAAATCCCCA
<210> 234
<211> 10
<212> DNA
<213> Homo sapiens
<400> 234

CCCGTCCGGA
<210> 235
<211> 10
<212> DNA
<213> Homo sapiens
<400> 235
TGGCTCCTCC
<210> 236
<211> 10
<212> DNA
<213> Homo sapiens
<400> 236
CTGACCTGTG
<210> 237
<211> 10
<212> DNA
<213> Homo sapiens
<400> 237
GGGGCAACAG
<210> 238
<211> 10
<212> DNA
<213> Homo sapiens
<400> 238
CACAAACGGT
<210> 239
<211> 10
<212> DNA
<213> Homo sapiens
<400> 239

GCGACCGTCA
<210> 240
<211> 10
<212> DNA
<213> Homo sapiens
<400> 240
CGCCGCCGGC
<210> 241
<211> 10
<212> DNA
<213> Homo sapiens
<400> 241
GTGCTGAATG
<210> 242
<211> 10
<212> DNA
<213> Homo sapiens
<400> 242
CCAGAACAGA
<210> 243
<211> 10
<212> DNA
<213> Homo sapiens
<400> 243
ACCGCCGTGG
<210> 244
<211> 10
<212> DNA
<213> Homo sapiens
<400> 244

GGAGGTGGGG
<210> 245
<211> 10
<212> DNA
<213> Homo sapiens
<400> 245
ACCCTTGGCC
<210> 246
<211> 10
<212> DNA
<213> Homo sapiens
<400> 246
TGCACGTTTT
<210> 247
<211> 10
<212> DNA
<213> Homo sapiens
<400> 247
CTGGGCCTGG
<210> 248
<211> 10
<212> DNA
<213> Homo sapiens
<400> 248
CCTCAGGATA
<210> 249
<211> 10
<212> DNA
<213> Homo sapiens
<400> 249

CGCCGACGAT
<210> 250
<211> 10
<212> DNA
<213> Homo sapiens
<400> 250
GTTCACATTA
<210> 251
<211> 10
<212> DNA
<213> Homo sapiens
<400> 251
TCCAAATCGA
<210> 252
<211> 10
<212> DNA
<213> Homo sapiens
<400> 252
AGGCTACGGA
<210> 253
<211> 10
<212> DNA
<213> Homo sapiens
<400> 253
AAAACATTCT
<210> 254
<211> 10
<212> DNA
<213> Homo sapiens
<400> 254

GGGCTGGGGT
<210> 255
<211> 10
<212> DNA
<213> Homo sapiens
<400> 255
TGCCTGCACC
<210> 256
<211> 10
<212> DNA
<213> Homo sapiens
<400> 256
GAAGCAGGAC
<210> 257
<211> 10
<212> DNA
<213> Homo sapiens
<400> 257
TGGTGTTGAG
<210> 258
<211> 10
<212> DNA
<213> Homo sapiens
<400> 258
ACACAGCAAG
<210> 259
<211> 10
<212> DNA
<213> Homo sapiens
<400> 259

TGTACCTGTA
<210> 260
<211> 10
<212> DNA
<213> Homo sapiens
<400> 260
TGGCCCCACC
<210> 261
<211> 10
<212> DNA
<213> Homo sapiens
<400> 261
CACCTCCTAT
<210> 262
<211> 10
<212> DNA
<213> Homo sapiens
<400> 262
CTGGGTTAAT
<210> 263
<211> 10
<212> DNA
<213> Homo sapiens
<400> 263
CCGTCCAAGG
<210> 264
<211> 10
<212> DNA
<213> Homo sapiens
<400> 264

GGATTTGGCC
<210> 265
<211> 10
<212> DNA
<213> Homo sapiens
<400> 265
GTGACCACGG
<210> 266
<211> 10
<212> DNA
<213> Homo sapiens
<400> 266
GCCGAGGAAG
<210> 267
<211> 10
<212> DNA
<213> Homo sapiens
<400> 267
ACCACTTATC
<210> 268
<211> 10
<212> DNA
<213> Homo sapiens
<400> 268
AGTTTCTTGT
<210> 269
<211> 10
<212> DNA
<213> Homo sapiens
<400> 269

AGCACCTCCA
<210> 270
<211> 10
<212> DNA
<213> Homo sapiens
<400> 270
GTGAAACCCT
<210> 271
<211> 10
<212> DNA
<213> Homo sapiens
<400> 271
GTGCGCTGAG
<210> 272
<211> 10
<212> DNA
<213> Homo sapiens
<400> 272
GTGCACTGAG
<210> 273
<211> 10
<212> DNA
<213> Homo sapiens
<400> 273
GGGAAGCAGA
<210> 274
<211> 10
<212> DNA
<213> Homo sapiens
<400> 274

CTGCCAAGTT
<210> 275
<211> 10
<212> DNA
<213> Homo sapiens
<400> 275
CGCAGTGTCC
<210> 276
<211> 10
<212> DNA
<213> Homo sapiens
<400> 276
TCGAAGCCCC
<210> 277
<211> 10
<212> DNA
<213> Homo sapiens
<400> 277
TTGGGTTTCC
<210> 278
<211> 10
<212> DNA
<213> Homo sapiens
<400> 278
ATAATTCTTT
<210> 279
<211> 10
<212> DNA
<213> Homo sapiens
<400> 279

CCTTGGGTTC
<210> 280
<211> 10
<212> DNA
<213> Homo sapiens
<400> 280
GTGGCCACGG
<210> 281
<211> 10
<212> DNA
<213> Homo sapiens
<400> 281
AAGACAGTGG
<210> 282
<211> 10
<212> DNA
<213> Homo sapiens
<400> 282
AGCAGATCAG
<210> 283
<211> 10
<212> DNA
<213> Homo sapiens
<400> 283
CTCATAAGGA
<210> 284
<211> 10
<212> DNA
<213> Homo sapiens
<400> 284

ATGGCTGGTA
<210> 285
<211> 10
<212> DNA
<213> Homo sapiens
<400> 285
AGGGCTTCCA
<210> 286
<211> 10
<212> DNA
<213> Homo sapiens
<400> 286
GCCGTGTCCG
<210> 287
<211> 10
<212> DNA
<213> Homo sapiens
<400> 287
CTGTTGGTGA
<210> 288
<211> 10
<212> DNA
<213> Homo sapiens
<400> 288
ACTAACACCC
<210> 289
<211> 10
<212> DNA
<213> Homo sapiens
<400> 289

GAGGGAGTTT
<210> 290
<211> 10
<212> DNA
<213> Homo sapiens
<400> 290
GGCAAGCCCC
<210> 291
<211> 10
<212> DNA
<213> Homo sapiens
<400> 291
CCCCCTGGAT
<210> 292
<211> 10
<212> DNA
<213> Homo sapiens
<400> 292
TAGGTTGTCT
<210> 293
<211> 10
<212> DNA
<213> Homo sapiens
<400> 293
TGGGCAAAGC
<210> 294
<211> 10
<212> DNA
<213> Homo sapiens
<400> 294

GGACCACTGA
<210> 295
<211> 10
<212> DNA
<213> Homo sapiens
<400> 295
GGCCGCGTTC
<210> 296
<211> 10
<212> DNA
<213> Homo sapiens
<400> 296
GTGAAGGCAG
<210> 297
<211> 10
<212> DNA
<213> Homo sapiens
<400> 297
AAGTTGCTAT
<210> 298
<211> 10
<212> DNA
<213> Homo sapiens
<400> 298
AGGAAAGCTG
<210> 299
<211> 10
<212> DNA
<213> Homo sapiens
<400> 299

CGCCGGAACA
<210> 300
<211> 10
<212> DNA
<213> Homo sapiens
<400> 300
CCCCAGTTGC
<210> 301
<211> 10
<212> DNA
<213> Homo sapiens
<400> 301
GTATGGGCCC
<210> 302
<211> 10
<212> DNA
<213> Homo sapiens
<400> 302
TGGCCCCAGG
<210> 303
<211> 10
<212> DNA
<213> Homo sapiens
<400> 303
CGACCCCACG
<210> 304
<211> 10
<212> DNA
<213> Homo sapiens
<400> 304

TAAATCCCCA
<210> 305
<211> 10
<212> DNA
<213> Homo sapiens
<400> 305
   AACGGGGCCC
<210> 306
<211> 10
<212> DNA
<213> Homo sapiens
<400> 306
TCACCGGTCA
<210> 307
<211> 10
<212> DNA
<213> Homo sapiens
<400> 307
GCCCCAGCCC
<210> 308
<211> 10
<212> DNA
<213> Homo sapiens
<400> 308
TGTCCCAGCC
<210> 309
<211> 10
<212> DNA
<213> Homo sapiens
<400> 309

CTGGACCCGG
<210> 310
<211> 10
<212> DNA
<213> Homo sapiens
<400> 310
CACCTCCTAT
<210> 311
<211> 10
<212> DNA
<213> Homo sapiens
<400> 311
AGAAGTGTCC
<210> 312
<211> 10
<212> DNA
<213> Homo sapiens
<400> 312
TGGCCCCAAG
<210> 313
<211> 10
<212> DNA
<213> Homo sapiens
<400> 313
ACATTCTTTT
<210> 314
<211> 10
<212> DNA
<213> Homo sapiens
<400> 314

ACTATTTCCA
<210> 315
<211> 10
<212> DNA
<213> Homo sapiens
<400> 315
CTCACCGCCC
<210> 316
<211> 10
<212> DNA
<213> Homo sapiens
<400> 316
TTATGGGGAG
<210> 317
<211> 10
<212> DNA
<213> Homo sapiens
<400> 317
GATGACCCCC
<210> 318
<211> 10
<212> DNA
<213> Homo sapiens
<400> 318
GCCATCCAGA
<210> 319
<211> 10
<212> DNA
<213> Homo sapiens
<400> 319

AATAGAAATT
<210> 320
<211> 10
<212> DNA
<213> Homo sapiens
<400> 320
AAGATTGGTG
<210> 321
<211> 10
<212> DNA
<213> Homo sapiens
<400> 321
GGTGGGGAGA
<210> 322
<211> 10
<212> DNA
<213> Homo sapiens
<400> 322
TCTTGATTTA
<210> 323
<211> 10
<212> DNA
<213> Homo sapiens
<400> 323
TTACAGAACT
<210> 324
<211> 10
<212> DNA
<213> Homo sapiens
<400> 324

ATGTGAAGAG
<210> 325
<211> 10
<212> DNA
<213> Homo sapiens
<400> 325
ACCTTTACTG
<210> 326
<211> 10
<212> DNA
<213> Homo sapiens
<400> 326
TCTCAGATGA
<210> 327
<211> 10
<212> DNA
<213> Homo sapiens
<400> 327
AAGGCGTTTC
<210> 328
<211> 10
<212> DNA
<213> Homo sapiens
<400> 328
GTGCTATTCT
<210> 329
<211> 10
<212> DNA
<213> Homo sapiens
<400> 329

CTTACAAGCA
<210> 330
<211> 10
<212> DNA
<213> Homo sapiens
<400> 330
GTGCTATTCT
<210> 331
<211> 10
<212> DNA
<213> Homo sapiens
<400> 331
TACCTGCAGA
<210> 332
<211> 10
<212> DNA
<213> Homo sapiens
<400> 332
TGGAAGCACT
<210> 333
<211> 10
<212> DNA
<213> Homo sapiens
<400> 333
CCCACAACCT
<210> 334
<211> 10
<212> DNA
<213> Homo sapiens
<400> 334

CTTGACATAC
<210> 335
<211> 10
<212> DNA
<213> Homo sapiens
<400> 335
TCTACACGTG
<210> 336
<211> 10
<212> DNA
<213> Homo sapiens
<400> 336
CTGATGGCGA
<210> 337
<211> 10
<212> DNA
<213> Homo sapiens
<400> 337
CTGTACTTGT
<210> 338
<211> 10
<212> DNA
<213> Homo sapiens
<400> 338
TGGAAAGTGA
<210> 339
<211> 10
<212> DNA
<213> Homo sapiens
<400> 339

TGAAGTAACA
<210> 340
<211> 10
<212> DNA
<213> Homo sapiens
<400> 340
ACATTTCCAA
<210> 341
<211> 10
<212> DNA
<213> Homo sapiens
<400> 341
ATGGTGGGGG
<210> 342
<211> 10
<212> DNA
<213> Homo sapiens
<400> 342
TACATTCTGT
<210> 343
<211> 10
<212> DNA
<213> Homo sapiens
<400> 343
TGGAGAAGAG
<210> 344
<211> 10
<212> DNA
<213> Homo sapiens
<400> 344

GGCCACGTAG
<210> 345
<211> 10
<212> DNA
<213> Homo sapiens
<400> 345
TTAACCCTCT
<210> 346
<211> 10
<212> DNA
<213> Homo sapiens
<400> 346
CTCCATCCAG
<210> 347
<211> 10
<212> DNA
<213> Homo sapiens
<400> 347
AGTGCACGTG
<210> 348
<211> 10
<212> DNA
<213> Homo sapiens
<400> 348
GGCCAGGACT
<210> 349
<211> 10
<212> DNA
<213> Homo sapiens
<400> 349

AGATGAGATG
<210> 350
<211> 10
<212> DNA
<213> Homo sapiens
<400> 350
TGAAGGATGC
<210> 351
<211> 10
<212> DNA
<213> Homo sapiens
<400> 351
CCCTGAGGCC
<210> 352
<211> 10
<212> DNA
<213> Homo sapiens
<400> 352
GTGGGCCACG
<210> 353
<211> 10
<212> DNA
<213> Homo sapiens
<400> 353
GGGAAACAGG
<210> 354
<211> 10
<212> DNA
<213> Homo sapiens
<400> 354

GTGGCCACGG
<210> 355
<211> 10
<212> DNA
<213> Homo sapiens
<400> 355
ACCATTCTGC
<210> 356
<211> 10
<212> DNA
<213> Homo sapiens
<400> 356
GCCGCCGTGC
<210> 357
<211> 10
<212> DNA
<213> Homo sapiens
<400> 357
CTTTTTTCCC
<210> 358
<211> 10
<212> DNA
<213> Homo sapiens
<400> 358
TGCACCACAG
<210> 359
<211> 10
<212> DNA
<213> Homo sapiens
<400> 359

GCAAAACCCT
<210> 360
<211> 10
<212> DNA
<213> Homo sapiens
<400> 360
TTGGAGCACT
<210> 361
<211> 10
<212> DNA
<213> Homo sapiens
<400> 361
TGGCCCCAGG
<210> 362
<211> 10
<212> DNA
<213> Homo sapiens
<400> 362
GTATGGGCCC
<210> 363
<211> 10
<212> DNA
<213> Homo sapiens
<400> 363
TAAATCCCCA
<210> 364
<211> 10
<212> DNA
<213> Homo sapiens
<400> 364

CGACCCCACG
<210> 365
<211> 10
<212> DNA
<213> Homo sapiens
<400> 365
CACCTCCTAT
<210> 366
<211> 10
<212> DNA
<213> Homo sapiens
<400> 366
ACCACTTATC
<210> 367
<211> 10
<212> DNA
<213> Homo sapiens
<400> 367
TCACCGGTCA
<210> 368
<211> 10
<212> DNA
<213> Homo sapiens
<400> 368
TGTCCCAGCC
<210> 369
<211> 10
<212> DNA
<213> Homo sapiens
<400> 369

TGGCCCCAAG
<210> 370
<211> 10
<212> DNA
<213> Homo sapiens
<400> 370
AATAGAAATT
<210> 371
<211> 10
<212> DNA
<213> Homo sapiens
<400> 371
ACCGACTGAT
<210> 372
<211> 10
<212> DNA
<213> Homo sapiens
<400> 372
CAGCTATTTC
<210> 373
<211> 10
<212> DNA
<213> Homo sapiens
<400> 373
TGGTTGAGCC
<210> 374
<211> 10
<212> DNA
<213> Homo sapiens
<400> 374

GATGACCCCC
<210> 375
<211> 10
<212> DNA
<213> Homo sapiens
<400> 375
AAGATTGGTG
<210> 376
<211> 10
<212> DNA
<213> Homo sapiens
<400> 376
GTCTGGGGGA
<210> 377
<211> 10
<212> DNA
<213> Homo sapiens
<400> 377
CCTTGGGTTT
<210> 378
<211> 10
<212> DNA
<213> Homo sapiens
<400> 378
GGGGCTTCTG
<210> 379
<211> 10
<212> DNA
<213> Homo sapiens
<400> 379

GATAGGATAA
<210> 380
<211> 10
<212> DNA
<213> Homo sapiens
<400> 380
CTAACTAGTT
<210> 381
<211> 10
<212> DNA
<213> Homo sapiens
<400> 381
TTAACCCCTC
<210> 382
<211> 10
<212> DNA
<213> Homo sapiens
<400> 382
CTCACCGCCC
<210> 383
<211> 10
<212> DNA
<213> Homo sapiens
<400> 383
TGTGGATGTG
<210> 384
<211> 10
<212> DNA
<213> Homo sapiens
<400> 384

GCATCCCTCC
<210> 385
<211> 10
<212> DNA
<213> Homo sapiens
<400> 385
   AGTAAAAGCT
<210> 386
<211> 10
<212> DNA
<213> Homo sapiens
<400> 386
CGGGATTCCT
<210> 387
<211> 10
<212> DNA
<213> Homo sapiens
<400> 387
CGAACCCACG
<210> 388
<211> 10
<212> DNA
<213> Homo sapiens
<400> 388
ACATTCTTTT
<210> 389
<211> 10
<212> DNA
<213> Homo sapiens
<400> 389

TGTTCATCAT
<210> 390
<211> 10
<212> DNA
<213> Homo sapiens
<400> 390
GCCCCAGCCC
<210> 391
<211> 10
<212> DNA
<213> Homo sapiens
<400> 391
TTTGGGCCTA
<210> 392
<211> 10
<212> DNA
<213> Homo sapiens
<400> 392
TGGGCAGCTG
<210> 393
<211> 10
<212> DNA
<213> Homo sapiens
<400> 393
CTGATGGCGA
<210> 394
<211> 10
<212> DNA
<213> Homo sapiens
<400> 394

GGCCAGGACT
<210> 395
<211> 10
<212> DNA
<213> Homo sapiens
<400> 395
GCAGAGAAAA
<210> 396
<211> 10
<212> DNA
<213> Homo sapiens
<400> 396
CTGTTGGCAT
<210> 397
<211> 10
<212> DNA
<213> Homo sapiens
<400> 397
CTGTACTTGT
<210> 398
<211> 10
<212> DNA
<213> Homo sapiens
<400> 398
CCCTGAGGCC
<210> 399
<211> 10
<212> DNA
<213> Homo sapiens
<400> 399

CTTGACATAC
<210> 400
<211> 10
<212> DNA
<213> Homo sapiens
<400> 400
GTGGGCCACG
<210> 401
<211> 10
<212> DNA
<213> Homo sapiens
<400> 401
ATCGCACCAC
<210> 402
<211> 10
<212> DNA
<213> Homo sapiens
<400> 402
ACATTTCCAA
<210> 403
<211> 10
<212> DNA
<213> Homo sapiens
<400> 403
TACCTGCAGA
<210> 404
<211> 10
<212> DNA
<213> Homo sapiens
<400> 404

ATGGTGGGGG
<210> 405
<211> 10
<212> DNA
<213> Homo sapiens
<400> 405
ACCATTCTGC
<210> 406
<211> 10
<212> DNA
<213> Homo sapiens
<400> 406
TACATTCTGT
<210> 407
<211> 10
<212> DNA
<213> Homo sapiens
<400> 407
GCCGCCGTGC
<210> 408
<211> 10
<212> DNA
<213> Homo sapiens
<400> 408
TGAAAACTAC
<210> 409
<211> 10
<212> DNA
<213> Homo sapiens
<400> 409

TGGAAGCACT
<210> 410
<211> 10
<212> DNA
<213> Homo sapiens
<400> 410
CCCACAACCT
<210> 411
<211> 10
<212> DNA
<213> Homo sapiens
<400> 411
GTGCCCGTGC
<210> 412
<211> 10
<212> DNA
<213> Homo sapiens
<400> 412
TTTCTGTATG
<210> 413
<211> 10
<212> DNA
<213> Homo sapiens
<400> 413
TAGCCCCCTG
<210> 414
<211> 10
<212> DNA
<213> Homo sapiens
<400> 414

TTAGGGAGGA
<210> 415
<211> 10
<212> DNA
<213> Homo sapiens
<400> 415
TGAACCCGGG
<210> 416
<211> 10
<212> DNA
<213> Homo sapiens
<400> 416
TAATGCTAAA
<210> 417
<211> 10
<212> DNA
<213> Homo sapiens
<400> 417
TTAACCCTCT
<210> 418
<211> 10
<212> DNA
<213> Homo sapiens
<400> 418
ACTACAAATA
<210> 419
<211> 10
<212> DNA
<213> Homo sapiens
<400> 419

GCGGCGGCTC
<210> 420
<211> 10
<212> DNA
<213> Homo sapiens
<400> 420
CATCTGTACT
<210> 421
<211> 10
<212> DNA
<213> Homo sapiens
<400> 421
CCTTTTTCAA
<210> 422
<211> 10
<212> DNA
<213> Homo sapiens
<400> 422
CAAATCCAAA
<210> 423
<211> 10
<212> DNA
<213> Homo sapiens
<400> 423
CTGACAGTGA
<210> 424
<211> 10
<212> DNA
<213> Homo sapiens
<400> 424

GCCTACCCGA
<210> 425
<211> 10
<212> DNA
<213> Homo sapiens
<400> 425
   AACGGGGCCC
<210> 426
<211> 10
<212> DNA
<213> Homo sapiens
<400> 426
CCCGCCTCTT
<210> 427
<211> 10
<212> DNA
<213> Homo sapiens
<400> 427
ACGGAACAAT
<210> 428
<211> 10
<212> DNA
<213> Homo sapiens
<400> 428
ATCGTGCGCT
<210> 429
<211> 10
<212> DNA
<213> Homo sapiens
<400> 429

CGCACCTCCA
<210> 430
<211> 10
<212> DNA
<213> Homo sapiens
<400> 430
CAGTTGCTAT
<210> 431
<211> 10
<212> DNA
<213> Homo sapiens
<400> 431
TAAGCAGGAC
<210> 432
<211> 10
<212> DNA
<213> Homo sapiens
<400> 432
GCCTGTCTGC
<210> 433
<211> 10
<212> DNA
<213> Homo sapiens
<400> 433
GCCGGCCGGA
<210> 434
<211> 10
<212> DNA
<213> Homo sapiens
<400> 434

ATGTGAAGAG
<210> 435
<211> 10
<212> DNA
<213> Homo sapiens
<400> 435
TTTGGGCCTA
<210> 436
<211> 10
<212> DNA
<213> Homo sapiens
<400> 436
CATCTGTACT
<210> 437
<211> 10
<212> DNA
<213> Homo sapiens
<400> 437
CCTGGGGTAA
<210> 438
<211> 10
<212> DNA
<213> Homo sapiens
<400> 438
TGGGAAATCG
<210> 439
<211> 10
<212> DNA
<213> Homo sapiens
<400> 439

ACCATCGTCC
<210> 440
<211> 10
<212> DNA
<213> Homo sapiens
<400> 440
CGGGTGAGCC
<210> 441
<211> 10
<212> DNA
<213> Homo sapiens
<400> 441
AGGGAGGGGC
<210> 442
<211> 10
<212> DNA
<213> Homo sapiens
<400> 442
TTCTCCCGCT
<210> 443
<211> 10
<212> DNA
<213> Homo sapiens
<400> 443
TCTGGTTTGT
<210> 444
<211> 10
<212> DNA
<213> Homo sapiens
<400> 444

GCGCGGGCGA
<210> 445
<211> 10
<212> DNA
<213> Homo sapiens
<400> 445
ATTCCTGAGC
<210> 446
<211> 10
<212> DNA
<213> Homo sapiens
<400> 446
GACTTGGCCT
<210> 447
<211> 10
<212> DNA
<213> Homo sapiens
<400> 447
ACTGCAGCCA
<210> 448
<211> 10
<212> DNA
<213> Homo sapiens
<400> 448
ATGGCGACTG
<210> 449
<211> 10
<212> DNA
<213> Homo sapiens
<400> 449

TATTGGCCTG
<210> 450
<211> 10
<212> DNA
<213> Homo sapiens
<400> 450
AGCAAATCCA
<210> 451
<211> 10
<212> DNA
<213> Homo sapiens
<400> 451
TTGGAACAAT
<210> 452
<211> 10
<212> DNA
<213> Homo sapiens
<400> 452
GGGGCTTCTG
<210> 453
<211> 10
<212> DNA
<213> Homo sapiens
<400> 453
TGATGTTTGA
<210> 454
<211> 10
<212> DNA
<213> Homo sapiens
<400> 454

TTGGCCCAAG
<210> 455
<211> 10
<212> DNA
<213> Homo sapiens
<400> 455
CTTTTTTCCC
<210> 456
<211> 10
<212> DNA
<213> Homo sapiens
<400> 456
CACTCGTGTG
<210> 457
<211> 10
<212> DNA
<213> Homo sapiens
<400> 457
CCTAACTGGA
<210> 458
<211> 10
<212> DNA
<213> Homo sapiens
<400> 458
GAGGGAGTCC
<210> 459
<211> 10
<212> DNA
<213> Homo sapiens
<400> 459

TGGGCTCTGA
<210> 460
<211> 10
<212> DNA
<213> Homo sapiens
<400> 460
TACCTGCAGA
<210> 461
<211> 10
<212> DNA
<213> Homo sapiens
<400> 461
CCGAAGGGTC
<210> 462
<211> 10
<212> DNA
<213> Homo sapiens
<400> 462
GGTTGGGGGC
<210> 463
<211> 10
<212> DNA
<213> Homo sapiens
<400> 463
TTTGTCTGTG
<210> 464
<211> 10
<212> DNA
<213> Homo sapiens
<400> 464

CCAAGAACAG
<210> 465
<211> 10
<212> DNA
<213> Homo sapiens
<400> 465
CATCTAAACT
<210> 466
<211> 10
<212> DNA
<213> Homo sapiens
<400> 466
GGGGGTTGGT
<210> 467
<211> 10
<212> DNA
<213> Homo sapiens
<400> 467
GTGCGTTGAG
<210> 468
<211> 10
<212> DNA
<213> Homo sapiens
<400> 468
CCCTTGGGTT
<210> 469
<211> 10
<212> DNA
<213> Homo sapiens
<400> 469

TCACCTTAGG
<210> 470
<211> 10
<212> DNA
<213> Homo sapiens
<400> 470
ATGATGCGGT
<210> 471
<211> 10
<212> DNA
<213> Homo sapiens
<400> 471
GGGTTGGGGT
<210> 472
<211> 10
<212> DNA
<213> Homo sapiens
<400> 472
CCTGTTATCC
<210> 473
<211> 10
<212> DNA
<213> Homo sapiens
<400> 473
GCTACAGTGA
<210> 474
<211> 10
<212> DNA
<213> Homo sapiens
<400> 474

GTGACAACAC
<210> 475
<211> 10
<212> DNA
<213> Homo sapiens
<400> 475
GTCACCTGCA
<210> 476
<211> 10
<212> DNA
<213> Homo sapiens
<400> 476
CGCACAATCA
<210> 477
<211> 10
<212> DNA
<213> Homo sapiens
<400> 477
GTTATAATAC
<210> 478
<211> 10
<212> DNA
<213> Homo sapiens
<400> 478
GCCCCAGGTA
<210> 479
<211> 10
<212> DNA
<213> Homo sapiens
<400> 479

GGTCAGTCGG
<210> 480
<211> 10
<212> DNA
<213> Homo sapiens
<400> 480
CTTCAGGATA

## Description

### Technical Field

The present invention of the application relates to gene groups expressed in human monocytes and human macrophages. More specifically, the application relates to gene groups individually comprising 100 genes highly expressed in human monocytes and two types of macrophages at different differentiation processes, a group of genes varying at the expression levels following the differentiation of monocytes to macrophages, the individual cDNA groups of these gene groups, novel gene groups contained in these gene groups, and oligonucleotide groups for identifying these genes and the cDNAs thereof.

### Background Art

Monocytes and macrophages are differentiated from bone marrow hematopoietic stem cells and play extremely important roles in biological protection against pathogenic organisms, healing of wounds, vascular formation and various chronic inflammatory diseases (for example, glanuloma, fibrosis and athelogenesis). At the normal state, monocyteas are recruited into various organs and body cavities, where monocytes are differentiated into macrophages (J. Exp. Med. 152:581, 1980; Blood 67: 1257, 1986). During local pathogenic infection and inflammation, chemotactic cytokines (chemokine) and various peptides or non-peptide inflammatory mediators are topically generated to induce monocyte infiltration, where monocytes are differentiated into macrophages. Macrophages change its morphology and function in various organs and body cavities, so macrophages are given with various designated names (for example, Kupffer cells in liver, alveolar macrophages in lung and microglia cells in central nervous system and the like). However, the relationship between blood monocytes and macrophages in various tissues has not yet been established.

Monocytes and macrophages have various characteristic properties in common, including the phagocytosis through Fcγ receptor and β2 integrin of extraneous particles, the generation of inflammatory cytokines and the like. However, the similarity and difference between monocytes and various macrophages at molecular levels have not yet been elucidated. Hence, the process of the differentiation of monocytes into macrophages and the functions of various macrophages are absolutely not elucidated at all currently.

The elucidation of monocytes and macrophages at their molecular levels is very significant not only for the understanding of their physiological roles but also for the elucidation of essential biological phenomena such as oncogenesis and individual development or the development of novel means for the diagnosis and therapeutic treatment of the individual diseases, in which monocytes and macrophages play important roles.

### Disclosure of the Invention

The invention of the application has been achieved in such circumstances. It is a purpose of the invention to provide gene groups individually comprising 100 genes specifically expressed in monocytes and macrophages.

Additionally, it is a purpose of the application to provide cDNA groups individually comprising cDNAs of these 100 genes, and oligonucleotide groups for identifying the gene groups and the cDNA groups.

Still additionally, it is a purpose of the application to provide a gene group comprising 30 genes being expressed more in human macrophages than in human monocytes, a gene group comprising 30 genes being expressed more inversely in human monocytes than in human macrophages, and gene groups individually comprising 30 genes being expressed more in one type of macrophages than in the other type of macrophages in these two types of macrophages at different differentiation processes. It is also a purpose of the application to provide the cDNA groups of these gene groups, novel gene groups therein and oligonucleotide groups therefor.

As a first gene group of the invention to solve the problems, the application provides a human monocyte-expressed gene group comprising 100 genes expressed most among the genes expressed in human monocytes, wherein the cDNAs of the individual genes individually contain the nucleotide sequences of SQ ID Nos. 1 to 100 consecutive to the nucleotide sequence CATG closest to the polyA region. Additionally, the application provides a cDNA group comprising the individual cDNAs of the first gene group, and a novel gene group contained in the gene group, and proteins encoded by the individual genes of the novel gene group, and antibodies against the proteins. Still additionally, it is a preferable embodiment of the human monocyte-expressed gene group that the expression frequency of the gene encoding the cDNA containing the nucleotide sequence of SQ ID No. 1 is the highest, while the decreasing order of the expression frequencies of the remaining 99 genes is the order of SQ ID Nos. 2 to 100.

As a second gene group, the application provides a human macrophage-expressed gene group comprising 100 genes expressed most among genes expressed in human macrophages differentiated through granulocyte-macrophage colony stimulating factor (GM-CSF) from human monocytes, wherein the cDNAs of the individual genes individually contain the nucleotide sequences of SQ ID Nos. 101 to 200 consecutive to the nucleotide sequence CATG closest to the polyA region. Furthermore, the application provides a cDNA group comprising the individual cDNAs of the second gene group, and a novel gene group contained in the gene group, and additionally proteins encoded by the individual genes of the novel gene group and antibodies against the proteins. Still furthermore, it is a preferable embodiment of the GM-CSF-induced macrophages (described as GM-macrophages hereinafter)-expressed gene group that the expression frequency of the gene encoding the cDNA containing SQ ID No. 101 is the highest, while the decreasing order of the expression frequencies of the remaining 99 genes is the order of SQ ID Nos. 102 to 200.

As a third gene group, the application provides a human macrophage-expressed gene group comprising 100 genes expressed most among genes expressed in human macrophages differentiated through macrophage colony stimulating factor (M-CSF) from human monocytes, wherein the CDNAs of the individual genes individually contain the nucleotide sequences of SQ ID Nos. 201 to 300 consecutive to the nucleotide sequence CATG closest to the polyA region. Furthermore, the application provides a cDNA group comprising the individual cDNAs of the third gene group, and a novel gene group contained in the gene group, and additionally proteins encoded by the individual genes of the novel gene group and antibodies against the proteins. Still furthermore, it is a preferable embodiment of the M-CSF-induced macrophages (described as M-macrophages hereinafter)-expressed gene group that the expression frequency of the gene encoding the cDNA containing SQ ID No. 201 is the highest, while the decreasing order of the expression frequencies of the remaining 99 genes is the order of SQ ID Nos. 202 to 300.

The application still additionally provides oligonucleotide groups individually consisting of nucleotide sequences of SQ ID Nos. 1 to 100, 101 to 200 and 201 to 300, which are consecutive to the nucleotide sequence CATG.

As a fourth gene group, the application provides a human macrophage-expressed gene group comprising 30 genes expressed more in GM-macrophages than in human monocytes, wherein the cDNAs of the individual genes individually contain the nucleotide sequences of SQ ID Nos. 301 to 330 consecutive to the nucleotide sequence CATG closest to the polyA region. Furthermore, the application provides a cDNA group comprising the individual cDNAs of the fourth gene group, and a novel gene group contained in the gene group, and additionally proteins encoded by the individual genes of the novel gene group and antibodies against the proteins.

As a fifth gene group, the application provides a human monocyte-expressed gene group comprising 30 genes expressed more in human monocytes than in GM-macrophages, wherein the the cDNAs of the individual genes individually contain the nucleotide sequences of SQ ID Nos. 331 to 360 consecutive to the nucleotide sequence CATG closest to the polyA region. Furthermore, the application provides a cDNA group comprising the individual cDNAs of the fifth gene group, and a novel gene group contained in the gene group, and additionally proteins encoded by the individual genes of the novel gene group and antibodies against the proteins.

As a sixth gene group, the application provides a human macrophage-expressed gene group comprising 30 genes expressed more in M-macrophages than in human monocytes, wherein the cDNAs of the individual genes individually contain the nucleotide sequences of SQ ID Nos. 361 to 390 consecutive to the nucleotide sequence CATG closest to the polyA region. Furthermore, the application provides a cDNA group comprising the individual cDNAs of the sixth gene group, and a novel gene group contained in the gene group, and additionally proteins encoded by the individual genes of the novel gene group and antibodies against the proteins.

As a seventh gene group, the application provides a human monocyte-expressed gene group comprising 30 genes expressed more in human monocytes than in M-macrophages, wherein the cDNAs of the individual genes individually contain the nucleotide sequences of SQ ID Nos. 391 to 420 consecutive to the nucleotide sequence CATG closest to the polyA region. Furthermore, the application provides a cDNA group comprising the individual cDNAs of the seventh gene group, and a novel gene group contained in the gene group, and additionally proteins encoded by the individual genes of the novel gene group and antibodies against the proteins.

As an eighth gene group, the application provides a human macrophage-expressed gene group comprising 30 genes expressed more in GM-macrophages than in M-macrophages, wherein the cDNAs of the individual genes individually contain the nucleotide sequences of SQ ID Nos. 421 to 450 consecutive to the nucleotide sequence CATG closest to the polyA region. Furthermore, the application provides a cDNA group comprising the individual cDNAs of the eighth gene group, and a novel gene group contained in the gene group, and additionally proteins encoded by the individual genes of the novel gene group and antibodies against the proteins.

As a ninth gene group, the application provides a human macrophage-expressed gene group comprising 30 genes expressed more in M-macrophages than in GM-macrophages, wherein the cDNAs of the individual genes individually contain the nucleotide sequences of SQ ID Nos. 451 to 480 consecutive to the nucleotide sequence CATG closest to the polyA region. Furthermore, the application provides a cDNA group comprising the individual cDNAs of the ninth gene group, and a novel gene group contained in the gene group, and additionally proteins encoded by the individual genes of the novel gene group and antibodies against the proteins.

### Best Mode for Carrying out the Invention

The first, second and third gene groups provided by the application are individual groups of 100 genes being expressed more in human monocytes, GM-macrophages and M-macrophages and occupying upper positions in the decreasing order of the expression frequencies, and are identified by known SAGE [Serial Analysis of Gene Expression (Science 276: 1268, 1997; Cell 88: 243, 1997; Science 270: 484, 1995; Nature 389:300, 1997; USP 5,695,937)]. The SAGE method comprises determining each 10-bp DNA sequence (Tag) characteristically representing each cDNA prepared from a gene expressed in an arbitrary cell. By calculating the ratio of each Tag to all of the resulting Tag species, the expression frequency (copy number) of each gene can be determined. Up to now, the following gene expressions have been analyzed by using the SAGE method. G2 arrest in colorectal cancer irradiation (Molecular Cell 1:3-11, 1997); Oleate medium and Pip2q regulation (18-th International Conference on Yeast Genetics and Molecular Biology 107: 58, 1997); Rat embryo fibroblast cells (Oncogene 15: 1079-1085, 1997); p53 expression (Nature 389: 300, 1997); Yeast genome (Cell 88: 243, 1997); Gastrointestinal tumors (Science 276:1268, 1997).

The first gene group of the invention (human monocyte-expressed gene group) is a group of genes identified on the basis of the method described in the individual references and US Patent set forth above. More specifically, human monocytes were firstly isolated from peripheral blood by Lymphoprep; from the resulting monocytes mRNA were prepared, which was then used to synthetically prepare cDNA by using biotinylated oligo(dT). Such cDNA was digested with restriction endonuclease NlaIII; and the 3'-termini of the individual cDNAs were purified by using avidin-magnet beads. Because the cleavage site with NlaIII is 5'-CATG-3', the 5'-terminal sequences of the purified 3'-terminal cDNA fragments are all "CATG", with no exception. Then, the cDNAs were divided in two fractions; different linkers were conjugated to the individual 5' termini; and subsequently using restriction endonuclease BsmF1, the cDNAs were cleaved downstream 14 bp apart from the 3' termini of the linkers. All 14-bp DNA fragments commonly carried the sequence CATG at the 5' terminus, but the remaining 10-bp varied, depending on each cDNA. Thus, the 10-bp sequence serves as Tag for specifying cDNA. For cloning a set of these Tag species, the BsmF1-cleavage site was firstly blunt ended; after division into two fragments, the resulting two cDNA fragments were ligated together; by PCR using oligonucleotides for the individual linkers as primers, DNA was amplified. In such manner, a DNA fragment (Ditag) of a sequence complimentary to the linker sequence (5'-GGATG-3') and 5'-CATG-3' at both the termini is recovered, where the different Tag sequences are conjugated at their individual 3' termini. After the amplified DNA fragment was digested with NlaIII, each Ditag was ligated together to prepare a concatemer, which was then cloned in a vector pZero1.

By the aforementioned procedure, 57,560 Tag species were recovered from the cDNA expressed in human monocytes. From all these Tag species, then, 100 Tag species with larger copy numbers (meaning in other words that the expression frequency of the cDNA designated by such Tag is high) were selected and shown as SQ ID Nos. 1 to 100.

As shown in the above description, these Tag species of SQ ID Nos. 1 to 100 comprised 10 base pairs consecutively present to the nucleotide sequence CATG present at the closest position to the polyA region of the cDNA of the gene expressed in human monocytes. Thus, screening of an existing DNA data base and the like and detection of the nucleotide sequence CATG closest to the polyA region of the DNA sequence in case of cDNA of a known sequence, and matching with the 10 base pairs consecutive to the nucleotide sequence CATG enables the identification of cDNA (and the gene encoding the cDNA) designated by the Tag. Tables 1 and 2 show the Tag species of SQ ID Nos. 1 to 100 (column A), the genes identified from the results of the screening of the data base (GeneBank) based on each Tag (column B) and the registry numbers thereof in the data base (column C).

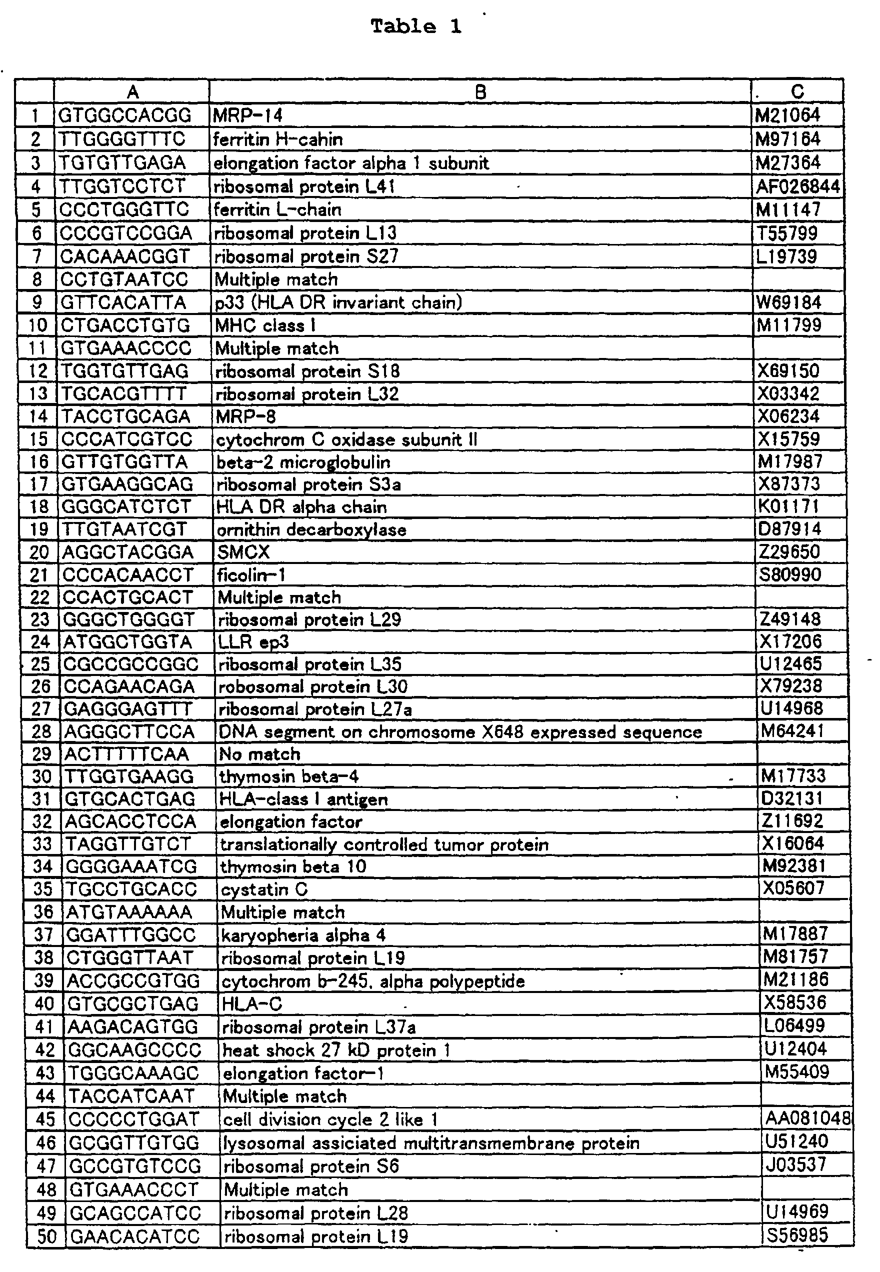

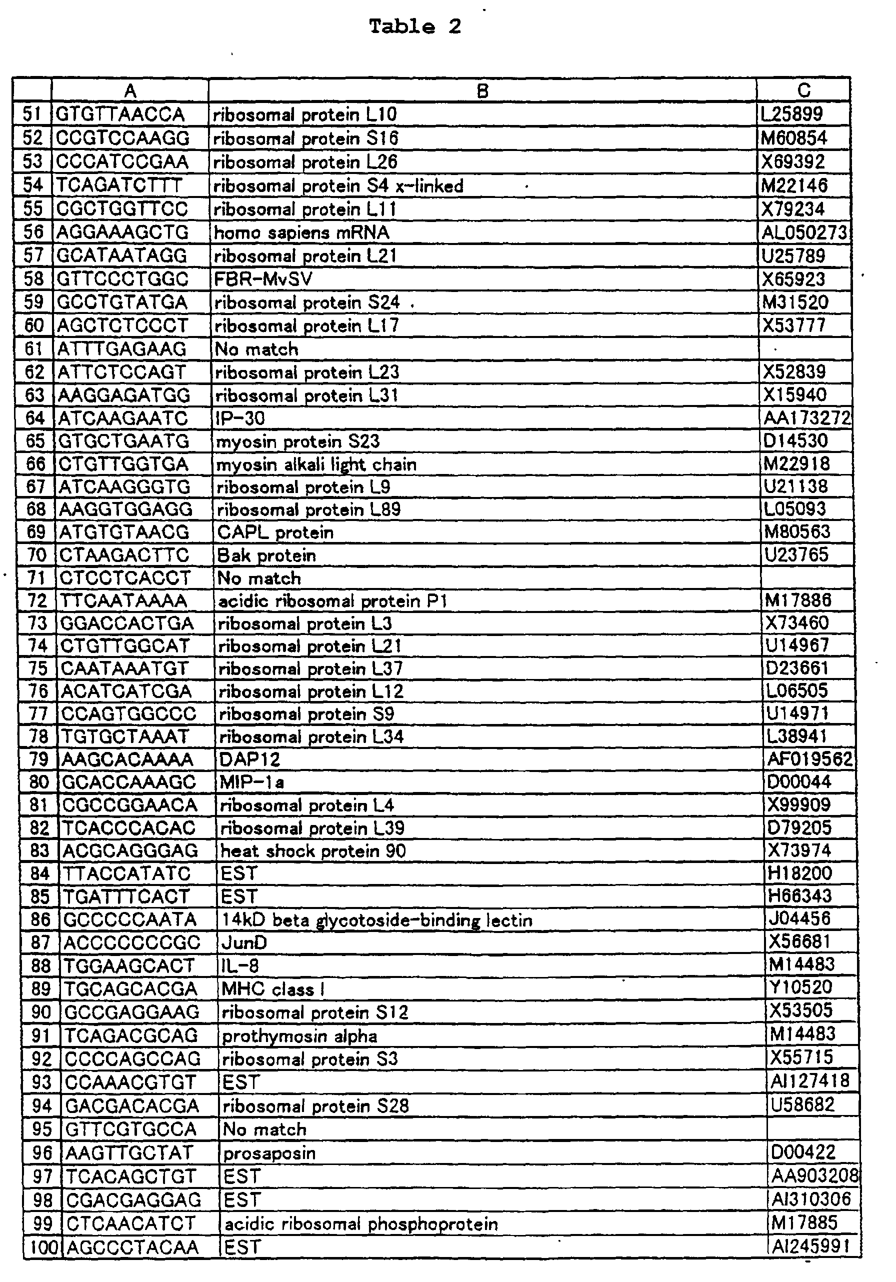

The second gene group (GM-macrophage-expressed gene group) and the third gene group (M-macrophage gene group) in accordance with the invention individually comprise a group of 100 genes occupying upper positions in the decreasing order of the expression frequencies, which were identified in the same manner as in the case of the first gene group (human monocyte-expressed gene group). More specifically, the GM-macrophage-expressed gene group is a group of genes designated by the Tag species of SQ ID Nos. 101 to 200, which were selected among all the 57,463 Tag species. The individual types are shown in Tables 3 and 4. Additionally, the M-macrophage-expressed gene group is a group of genes designated by the Tag species of SQ ID Nos. 201 to 300, which were selected among all the 55,856 Tag species. The individual types are shown in Tables 5 and 6.

Herein, it has been known that monocytes differentiated with GM-CSF and M-CSF show different morphology and functions (Blood 77:1131, 1991; J. Interferon Cytokine Res. 16:237, 1996; AIDS Res Hum Retroviruses 11: 1131, 1995; Cell 28:71, 1982).

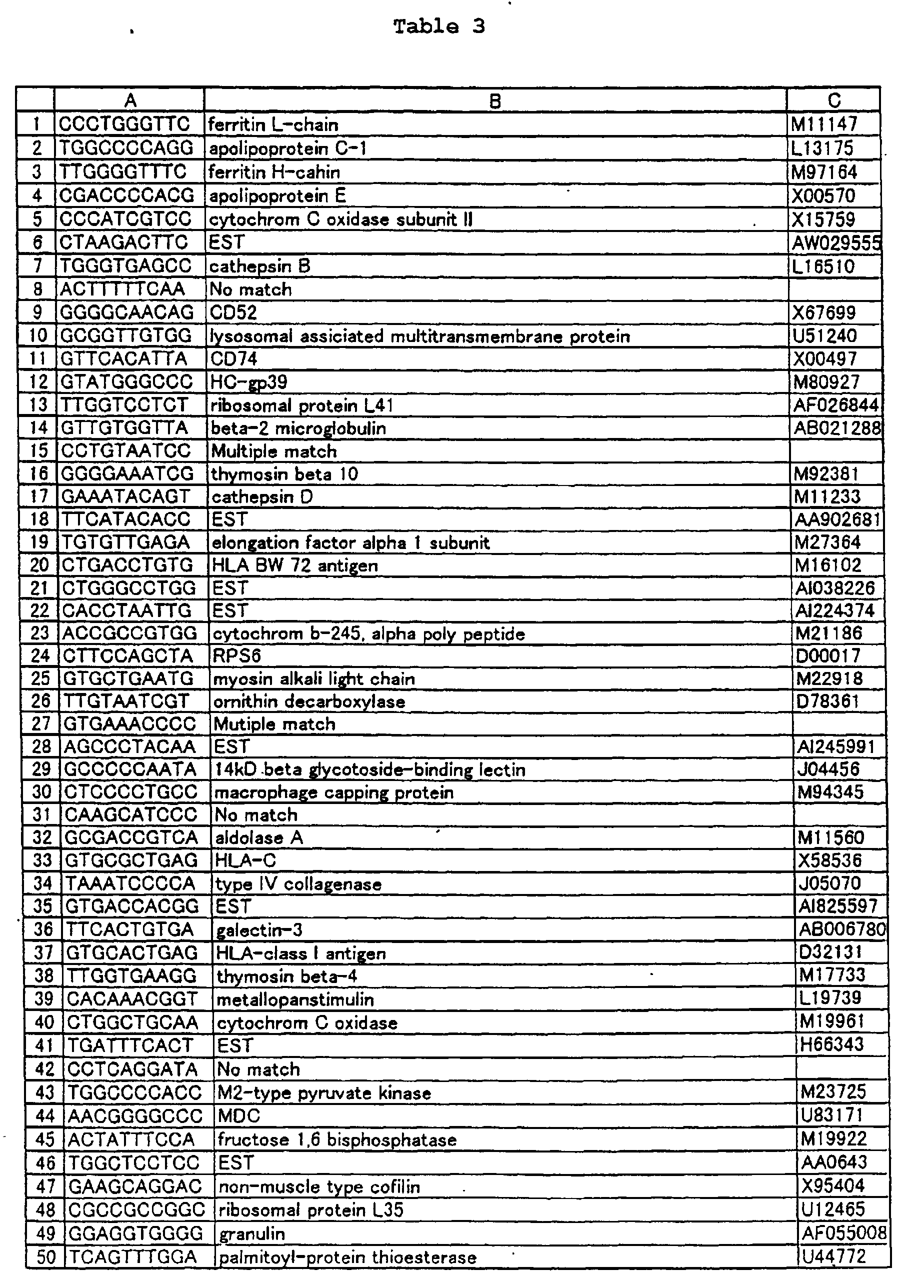

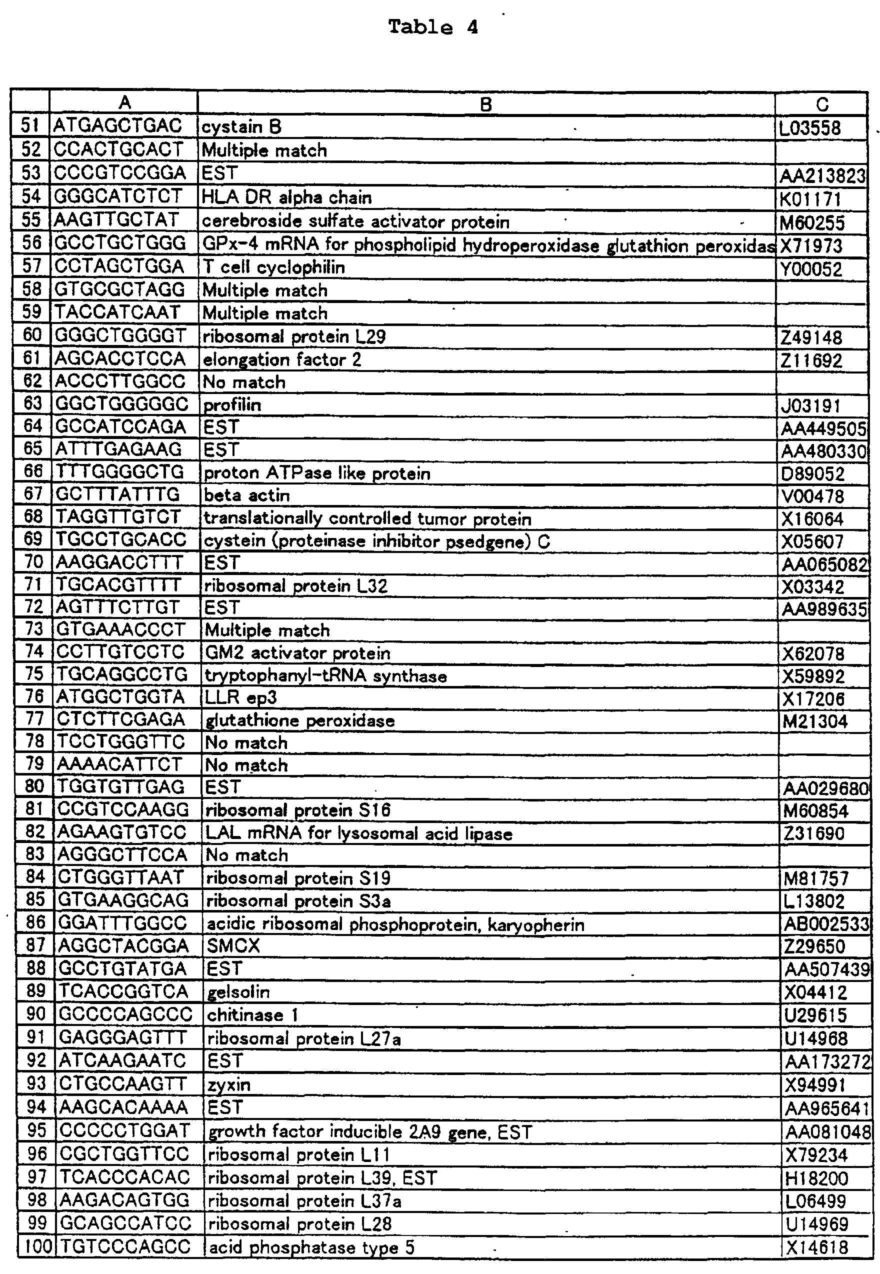

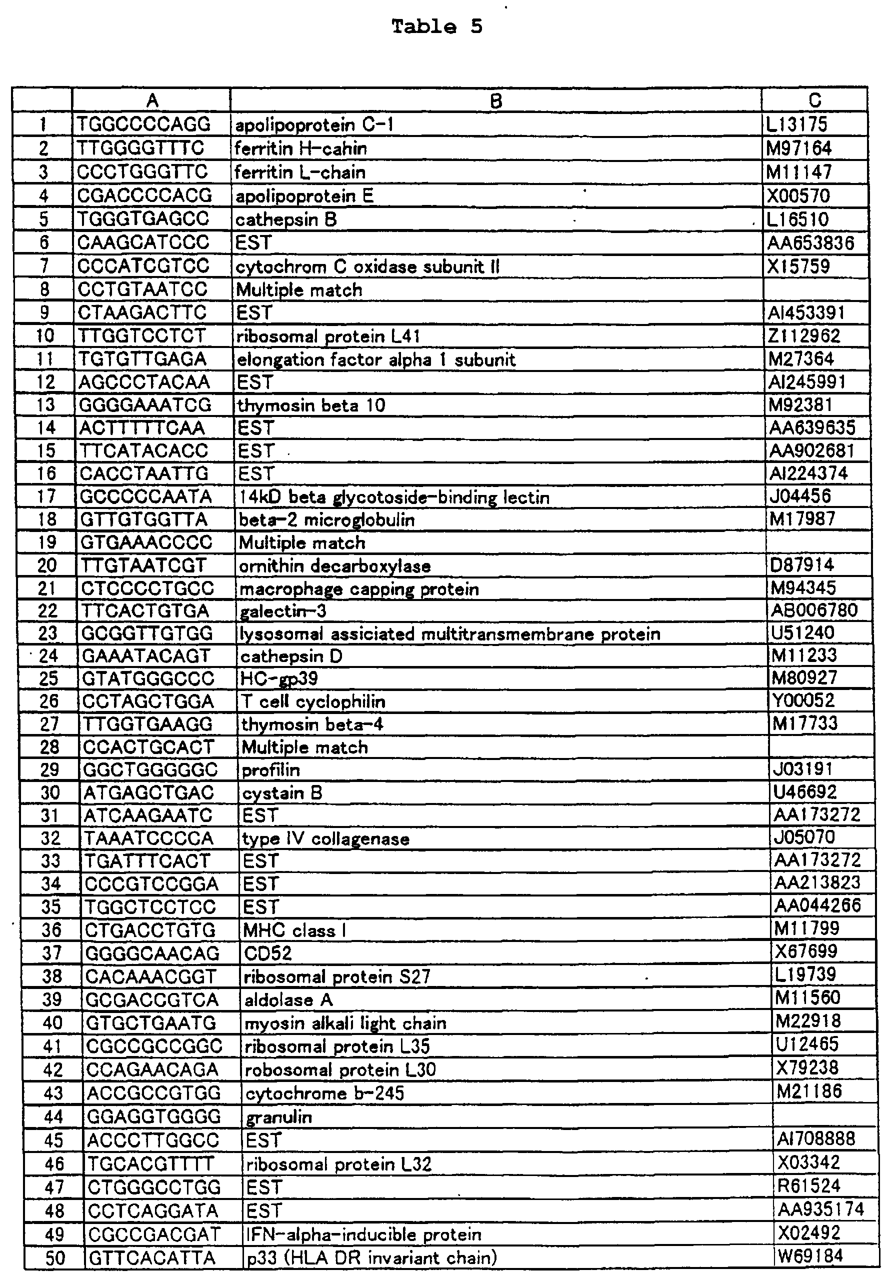

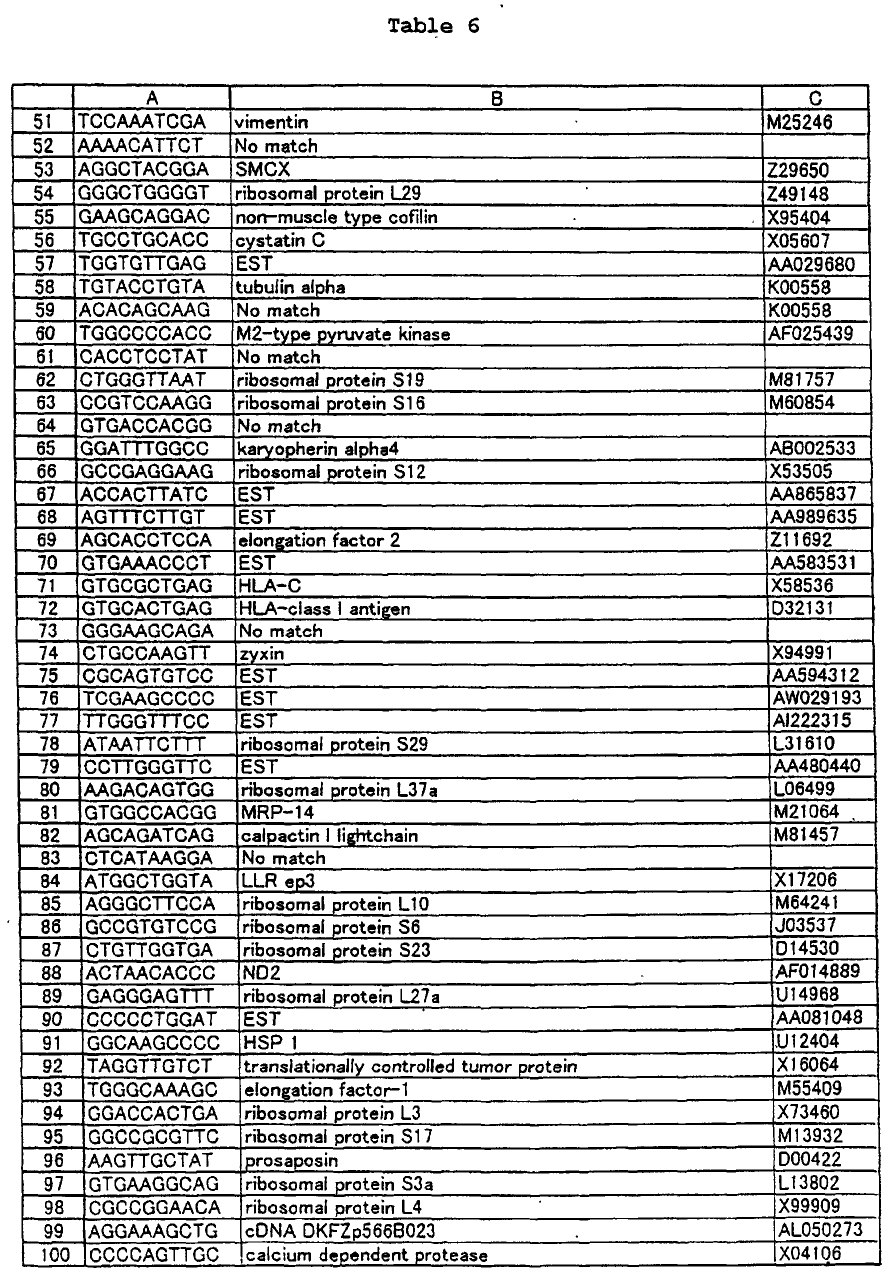

These lists in Tables 1 to 6 indicate for example the following findings. As shown in Tables 1 and 2, firstly, the gene expressed most in human monocyte is the MRP-14 gene and the expression frequency is 1.87 %. The individual genes of ferritin H-chain and elongation factor α- subunit follow the MRP-14 gene. Alternatively, the genes expressed most in GM-macrophage and M-macrophage are the genes of the ferritin L-chain (at expression frequency of 2.69 %) and apolipoprotein C-1 (at expression frequency of 2.21 %), respectively. Additionally, expression of vast amounts of genes encoding cell backbone proteins (for example, lipid metabolism-related proteins, mitochondria protein, protease and ion regulatory proteins and the like) was also observed.

The fourth gene group of the invention is a gene group comprising 30 genes occupying upper positions in the decreasing order of the expression frequencies, which are expressed more in GM-macrophages than in human monocytes. Concerning the 30 genes expressed more in GM-macrophages than in human monocytes, Table 7 shows the individual Tag species (SQ ID Nos. 301 to 330) and the copy number as well as the individual copy numbers thereof in the monocytes and M-macrophages. Herein, the left column of Table 7 indicates the ratio in fold of the copy number of the GM-macrophage-expressed gene to the copy number of the monocyte-expressed gene.

The genes at higher expression levels in GM-macrophages compared with the monocyte-expressed genes were also highly expressed in M-macrophages, with some exception. For example, the Tag frequency of hc-gp39 was 0 in monocytes, while the Tag frequencies thereof in GM-macrophages and M-macrophages were 288 and 182, respectively. Additionally, the expression levels of proteins with relation to lipid metabolism (for example, apolipoprotein E, osteopontin, CD9, sterol 27-hydroxylase, lisosomal acid lipase) were high in macrophages, compared with monocytes. This conforms with the finding that these lipid metabolism-related proteins have some relation with athelogenesis.

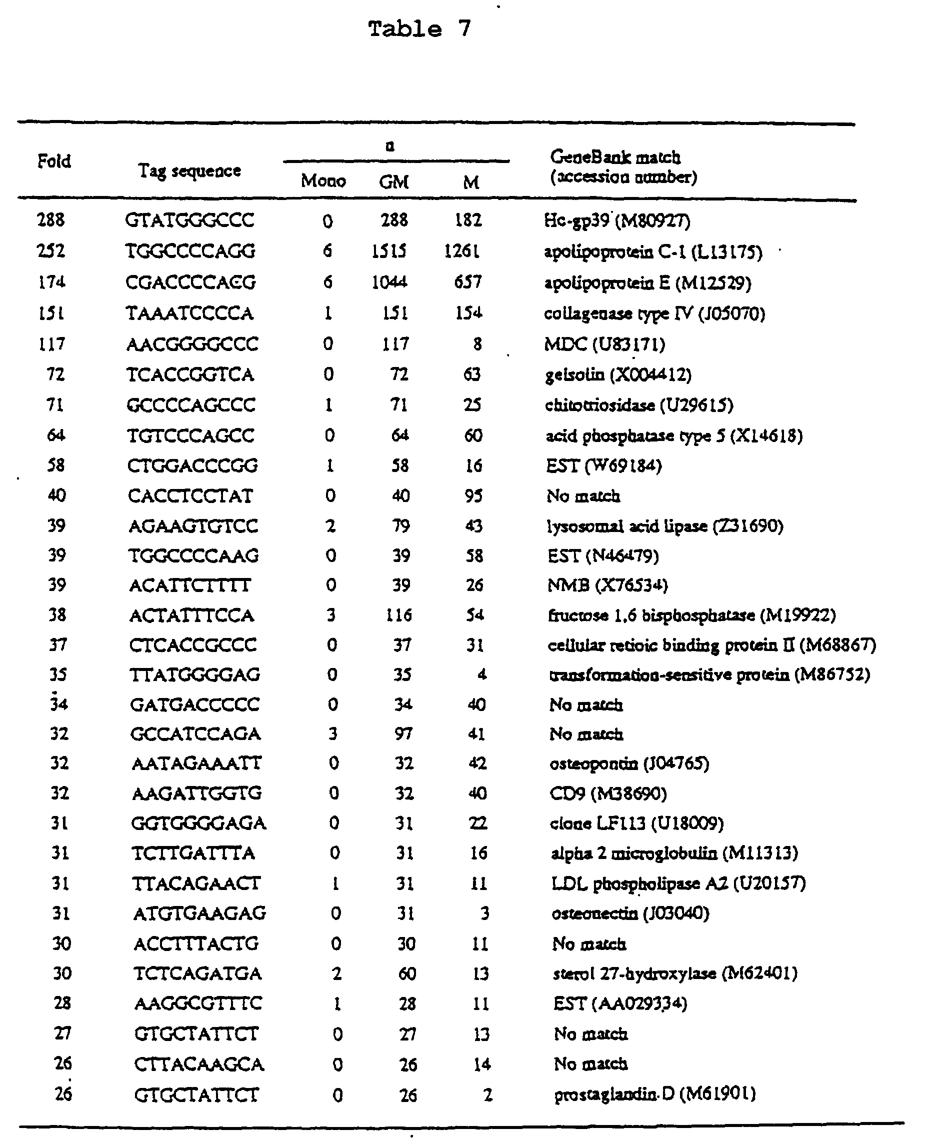

The fifth gene group of the invention is a gene group comprising 30 genes occupying upper positions in the decreasing order of the expression frequencies, which are expressed more in human monocytes than in GM-macrophages. Like Table 7, Table 8 shows these genes and the individual Tag species thereof (SQ ID Nos. 331 to 360). These genes exerted similar tendencies in GM- and M-macrophages. The genes at particularly reduced expression levels following the differentiation of monocytes into macrophages are genes of complement proteins (ficolin, properdin), DNA binding proteins (GOS3, GOS2), tristetraprolin and CPBP.

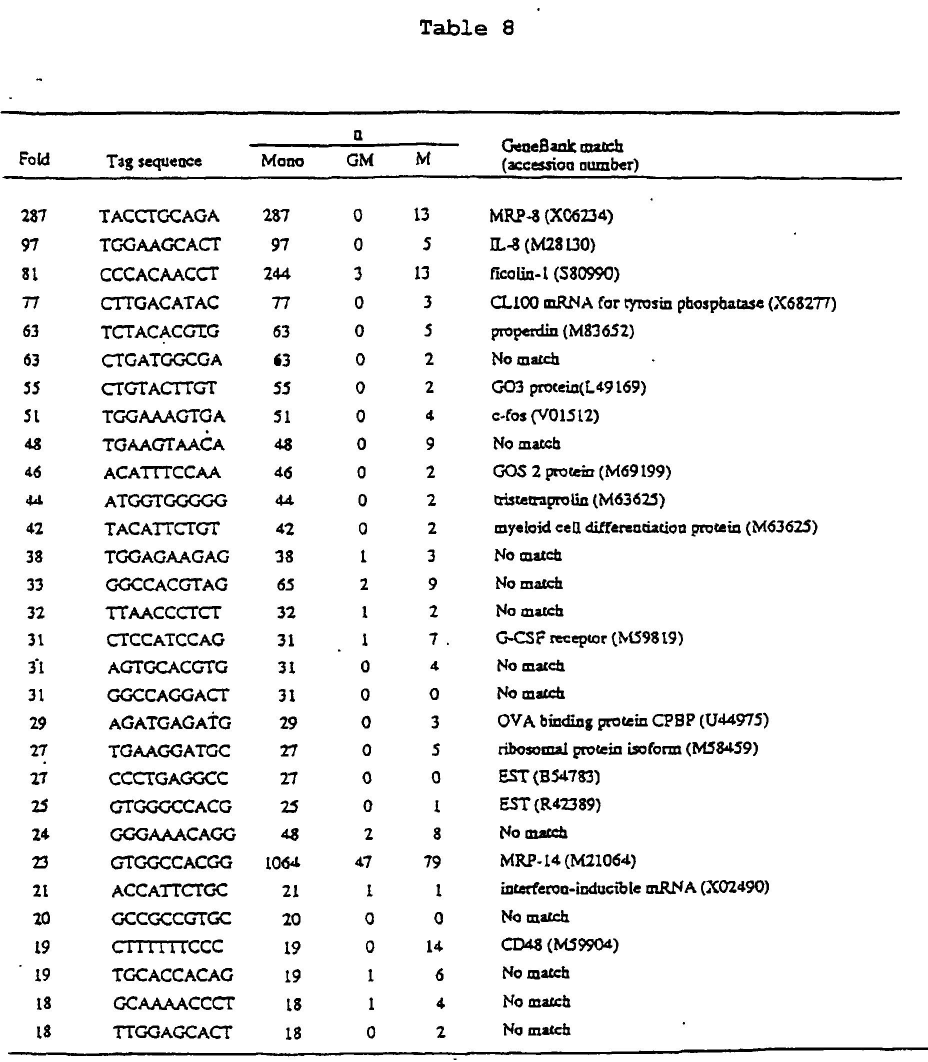

The sixth gene group of the invention is a gene group comprising 30 genes occupying upper positions in the decreasing order of the expression frequencies, which are expressed more in M-macrophages than in human monocytes. Table 9 shows the individual Tag species of SQ ID Nos. 361 to 390 (column A), genes identified from the results of the screening of data base (GeneBank and the like) based on the individual Tag species (column B) and the registry numbers thereof in the data base (column C).

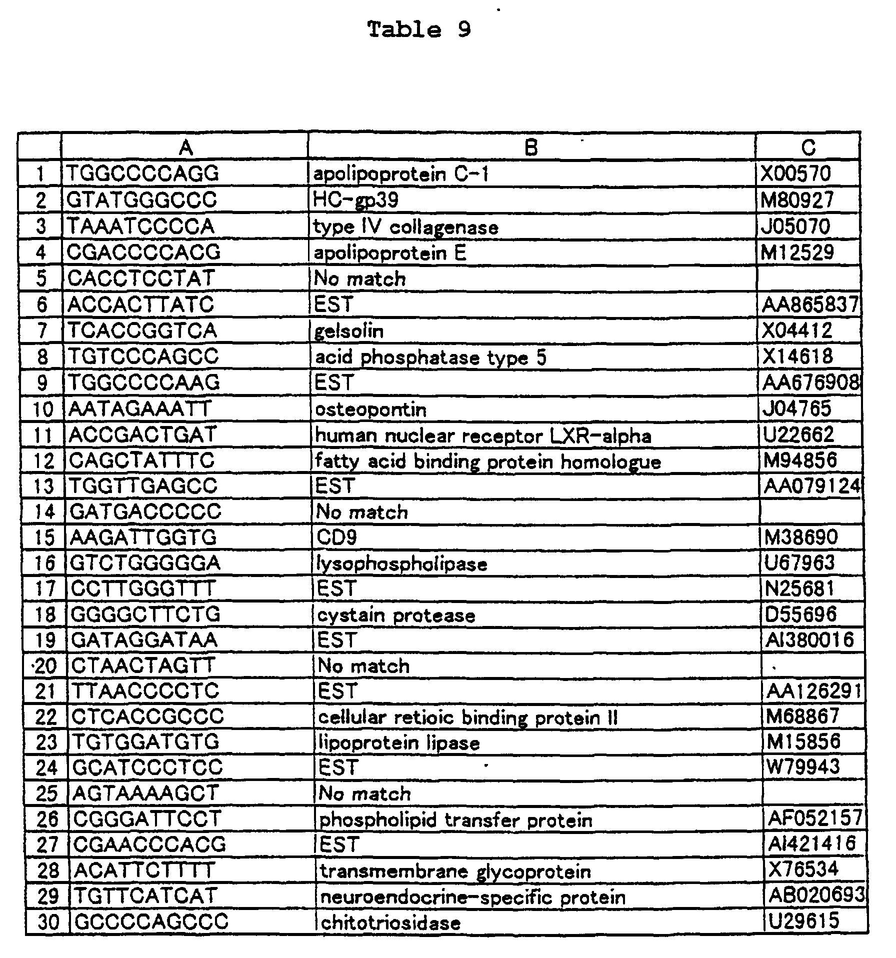

The seventh gene group of the invention is a gene group comprising 30 genes occupying upper positions in the decreasing order of the expression frequencies, which are expressed more in human monocytes than in M-macrophages. Like Table 9, Table 10 shows these genes and the individual Tag species (SQ ID Nos. 391 to 420) thereof.

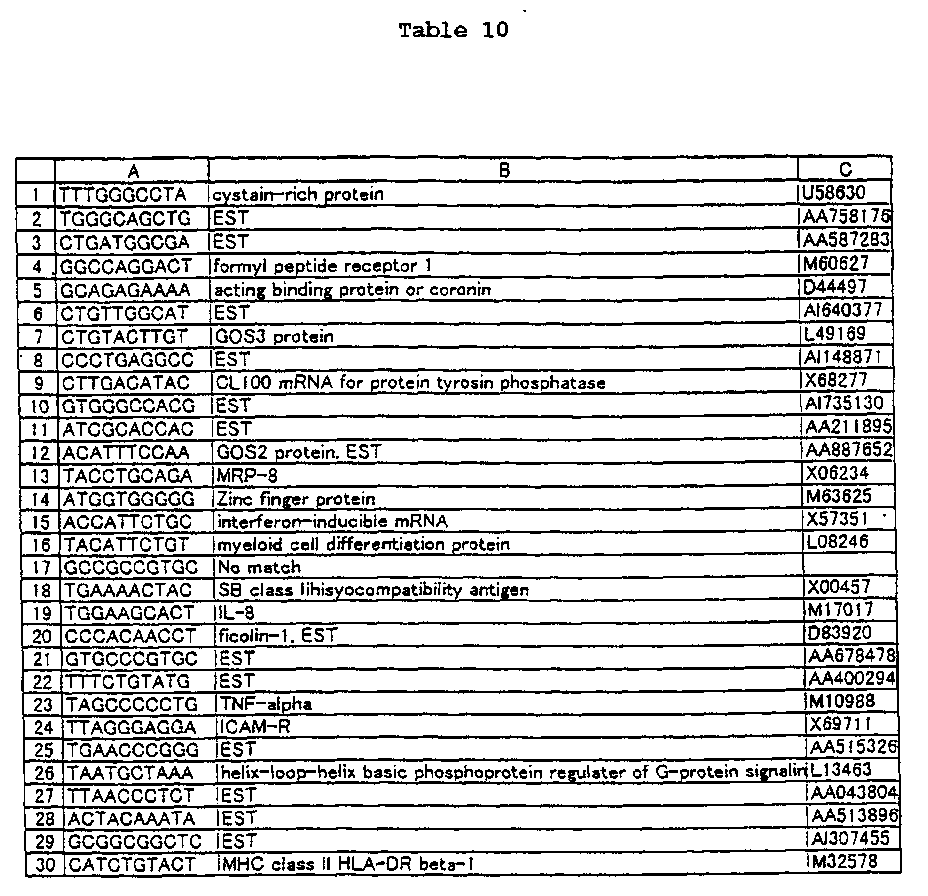

The eighth gene group of the invention is a gene group comprising 30 genes occupying upper positions in the decreasing order of the expression frequencies, which are expressed more in GM-macrophages than in M-macrophages. Additionally, the ninth gene group of the invention is a gene group comprising 30 genes occupying upper positions in the decreasing order of the expression frequencies, which are expressed more in M-macrophages than in GM-macrophages. These gene groups and the Tag species thereof (SQ ID Nos. 421 to 450, and 451 to 480) are individually shown in Tables 11 and 12. Additionally, Table 13 shows the copy numbers of the 15 genes of each gene group in monocytes, GM-macrophages and M-macrophages, which occupy upper positions. Based on these Tables, it is confirmed for example that MDC highly expressed in GM-macrophages is hardly expressed in M-macrophages. Because MDC is a novel chemokine and attracks selectively CCR4-positive Th2-type lymphocytes (J. Leukoc Biol. 64:49, 1998), it is thus indicated that the infiltration of GM-macrophages is responsible for Th2 immune diseases.

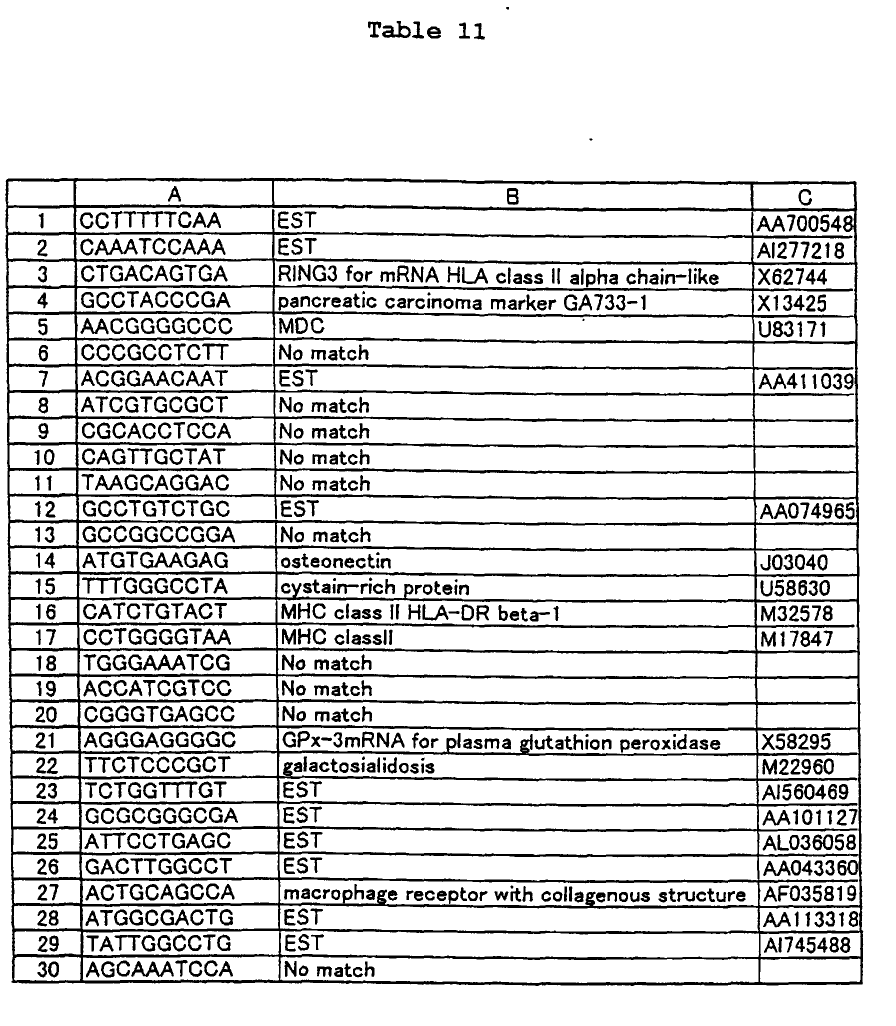

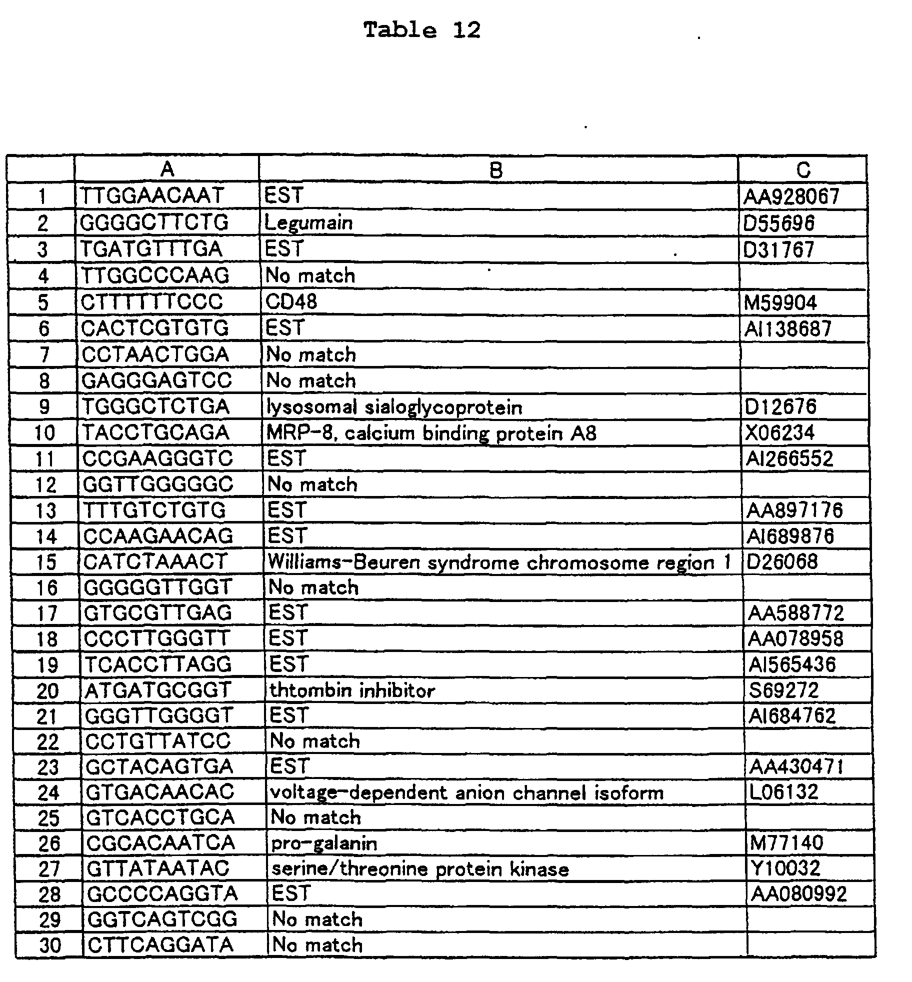

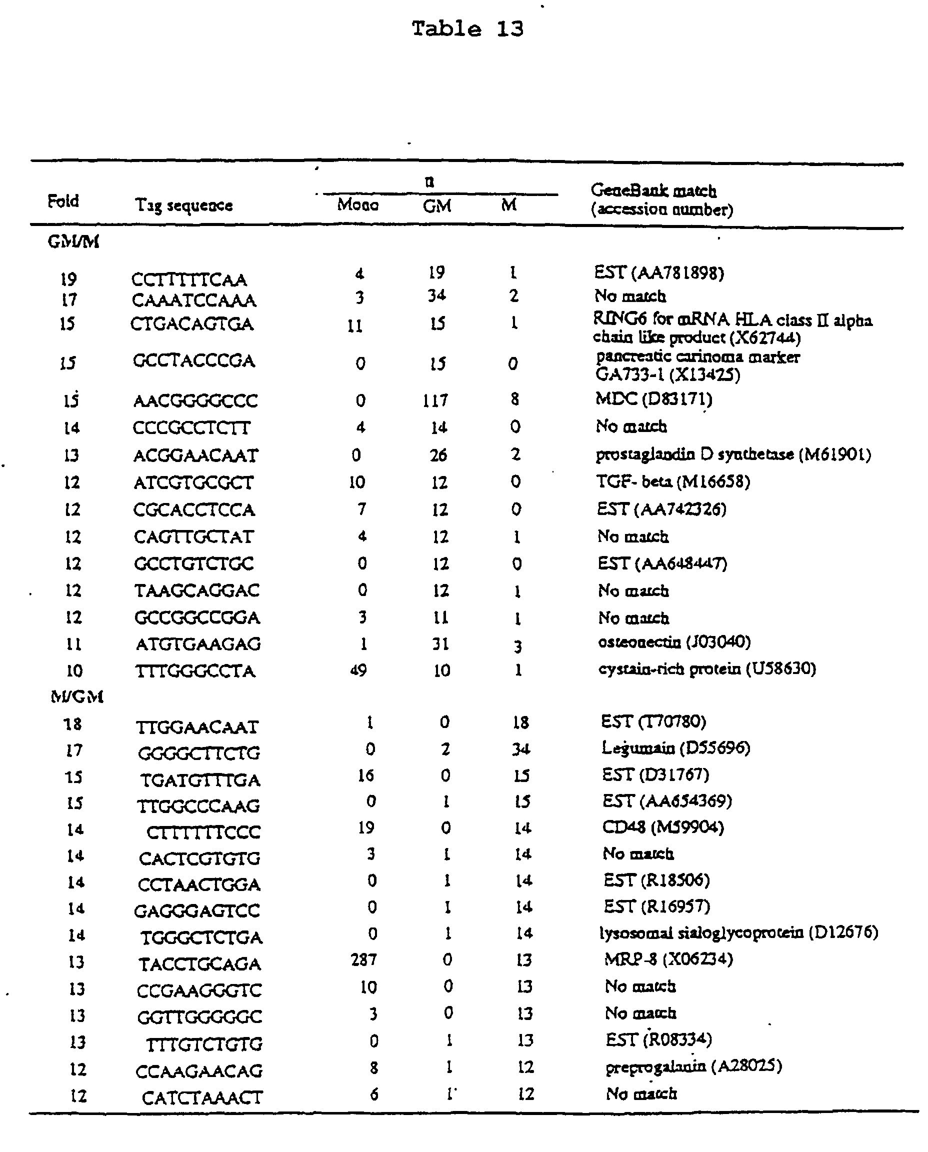

Furthermore, the cDNA groups of the application comprise the cDNAs of the genes composing the individual gene groups, wherein the cDNAs carry the nucleotide sequences of SQ ID Nos. 1 to 100, 101 to 200, 201 to 300, 301 to 330, 331 to 360, 361 to 390, 391 to 420, 421 to 450, and 451 to 480 consecutive to the nucleotide sequence CATG closest to the polyA region or a group of partial sequences thereof. Furthermore, human gene polymorphism due to individual difference is generally observed at high frequency. Thus, the inventive cDNA groups include cDNAs with addition or deletion of one or plural nucleotides and/or substitution with other nucleotides in the cDNA groups. Additionally, the inventive cDNA groups encompass DNA fragments (10 bp or more) carrying all partial nucleotide sequences of the cDNAs. Still more additionally, the cDNA groups include DNA fragments comprising sense chain and antisense chain.

As has been described above, the gene groups provided by the invention individually comprise 100 genes including novel genes, which are all expressed in human monocytes and macrophages differentiated through different differentiation pathways from monocytes and occupy the highest positions in the decreasing order of the expression frequencies. Additionally, the gene groups provided by the invention are gene groups at relatively high expression levels in human monocytes and human macrophages. Thus, these gene groups, the cDNA groups or the Tag species designating them are useful for example in the following fields.

### (1) Identification of novel genes

In accordance with the application, genes registered as "NO match" and "EST" in the column depicting the results of the database screening in Tables 1 to 12 are defined to belong to "novel gene group". "No match" expresses the case with no corresponding gene present as the term represents. Further, "EST" is included in novel gene because a partial sequence of the cDNA is disclosed but the function of the gene is not identified. These novel genes in the novel gene group can be identified by positional cloning using as marker oligonucleotides with CATG added to the 5' termini of the Tag sequences, screening of cDNA libraries using the oligonucleotides as probes or screening of gene banks. Additionally, novel proteins encoded by the novel genes and antibodies against the proteins can be recovered. The proteins can be recovered for example by an in vitro translation method of the cDNAs of the novel genes or partial sequenced thereof and a method for expressing the novel genes in appropriate host-vectors. Furthermore, the antibodies can be prepared as polyclonal antibodies or monoclonal antibodies according to known methods.

### (2) Identification of disease genes

The etiologic genes of diseases in which human monocytes and macrophages are involved (chronic inflammations such as granuloma, fibrosis, and athelogenesis) are likely expressed at high levels. For example, excess expression of specific genes included in the individual gene groups provided by the invention, decrease of the expression levels thereof, deletion thereof or gene mutation or the like is listed. As described above, furthermore, genes with apparently different expression levels in between monocytes and the two macrophages are present. Thus, these genes at different expression levels are individually potent candidates for the etiologic genes of the diseases responsible for cell functions. As has been described above, the individual gene groups provided by the invention are useful for the elucidation of these disease genes and the onset mechanisms of the diseases.

### (3) Development of pharmaceutical agents

The elucidation of the etiologic genes and the onset mechanisms as described above in 2 is useful for the development of therapeutic pharmaceutical agents for the diseases. The therapeutic pharmaceutical agents include for example pharmaceutical agents reducing the expression levels of excessively expressed genes.

### (4) Development of diagnostic means

The invention is useful for the development of diagnostic means of diseases in which human monocytes and macrophages are involved. For example, kits for EIA and RIA utilizing antibodies against proteins expressed by the etiologic genes or DNA chips with parts of the genes or the entire lengths thereof integrated therein are listed. In particular, DNA chips are useful for the diagnosis of diseases for which plural genes are responsible. Because the number of DNA fragments to be possibly integrated on a substrate, however, genes required for such diagnosis are necessarily selected. The invention provides genes at higher expression levels and cDNAs thereof. Thus, it can absolutely be said that these belong to a group of genes playing important roles in cellular functions and the cDNAs thereof. Therefore, these are extremely useful as DNA candidates composing DNA chips. Additionally, the oligonucleotide groups including the Tag sequences in accordance with the invention comprise sequences accurately identifying the individual genes. Therefore, the oligonucleotide groups can be utilized as minimum DNA sequences to be incorporated in the DNA chips.

### Industrial Applicability

As has been described above in detail, the application provides gene groups individually comprising 100 genes highly expressed in human monocytes and two types of macrophages at different differentiation processes. These gene groups are exceedingly useful not only for the understanding of the physiological functions of monocytes and macrophages but also the elucidation of essential biological phenomena such as oncogenesis and individual development or the development of novel means for the diagnosis and therapeutic treatment of the individual diseases where monocytes and macrophages play important roles.

## Claims

1. A human monocyte-expressed gene group comprising 100 genes at the highest expression frequency among genes expressed in human monocytes, wherein the cDNAs off the individual genes individually contain nucleotide sequences of SQ ID Nos. 1 to 100 consecutive to the nucleotide sequence CATG closest to the polyA region.

2. A human monocyte-expressed gene group according to claim 1, wherein the expression frequency of the gene encoding the cDNA, containing the nucleotide sequence of SQ ID No. 1 is the highest and the expression frequencies of the remaining 99 genes are in the order of SQ ID Nos. 2 to 100.

3. A human monocyte-expressed gene cDNA group comprising the cDNAs or partial sequences thereof of the individual genes composing a human monocyte-expressed gene group according to claim 1, wherein the cDNAs individually contains nucleotide sequences of SQ ID Nos. 1 to 100 consecutive to the nucleotide sequence CATG closest to the polyA region.

4. A human monocyte-expressed novel gene group included in a human monocyte-expressed gene group according to claim 1, wherein the cDNAs of the individual novel genes individually contain nucleotide sequences of SQ ID Nos. 29, 61, 71, 84, 85, 93, 95, 97, 98 and 100 consecutive to the nucleotide sequence CATG closest to the polyA region.

5. Proteins encoded by the individual genes of a human monocyte-expressed novel gene group according to claim 4, and antibodies against the proteins.

6. A group of oligonucleotides individually consisting of nucleotide sequences of SQ ID Nos. 1 to 100 consecutive to the nucleotide sequence CATG.

7. A human macrophage-expressed gene group comprising 100 genes at the highest expression frequency among genes expressed in human macrophages differentiated through granulocyte-macrophage colony stimulating factor from human monocytes, wherein the cDNAs of the individual genes individually contain nucleotide sequences of SQ ID Nos. 101 to 200 consecutive to the nucleotide sequence CATG closest to the polyA region.

8. A human macrophage-expressed gene group according to claim 7, wherein the expression frequency of the gene encoding the cDNA containing the nucleotide sequence of SQ ID No. 101 is the highest and the expression frequencies of the remaining 99 genes are in the order of SQ ID Nos. 102 to 200.

9. A human macrophage-expressed gene cDNA group comprising the cDNAs or partial sequences thereof of the individual genes composing a human macrophage-expressed gene group according to claim 7, wherein the cDNAs individually contain nucleotide sequences of SQ ID Nos. 101 to 200 consecutive to the nucleotide sequence CATG closest to the polyA region.

10. A human macrophage-expressed novel gene group included in a human macrophage-expressed gene group according to claim 7, wherein the cDNAs of the individual novel genes individually contain nucleotide sequences of SQ ID Nos. 106, 108, 118, 121, 122, 128, 131, 135, 141, 142, 146, 153, 162, 164, 165, 170, 172, 178 to 180, 183, 188, 192 and 194.

11. Proteins encoded by the individual genes of a human macrophage-expressed novel gene group according to claim 10, and antibodies against the proteins.

12. A group of oligonucleotides individually consisting of nucleotide sequences of SQ ID Nos. 101 to 200 consecutive to the nucleotide sequence CATG.

13. A human macrophage-expressed gene group comprising 100 genes at the highest expression frequency among genes expressed in human macrophages differentiated through macrophage colony stimulating factor from human monocytes, wherein the cDNAs of the individual genes individually contain nucleotide sequences of SQ ID Nos. 201 to 300 consecutive to the nucleotide sequence CATG closest to the polyA region.

14. A human macrophage-expressed gene group according to claim 13, wherein the expression frequency of the gene encoding the cDNA containing the nucleotide sequence of SQ ID No. 201 is the highest and the expression frequencies of the remaining 99 genes are in the order of SQ ID Nos. 202 to 300.

15. A human macrophage-expressed gene cDNA group comprising the cDNAs or partial sequences thereof of the individual genes composing a human macrophage-expressed gene group according to claim 13, wherein the cDNAs individually contain nucleotide sequences of SQ ID Nos. 201 to 300 consecutive to the nucleotide sequence CATG closest to the polyA region.

16. A human macrophage-expressed novel gene group included in a human macrophage-expressed gene group according to claim 13, wherein the cDNAs of the individual novel genes individually contain nucleotide sequences of SQ ID Nos. 206, 209, 212, 214 to 216, 231, 233 to 235, 245, 247, 248, 252, 257, 259, 261, 264, 267, 268, 270, 273, 275 to 277, 279, 283 and 290.

17. Proteins encoded by the individual genes of a human macrophage-expressed novel gene group according to claim 16, and antibodies against the proteins.

18. A group of oligonucleotides individually consisting of nucleotide sequences of SQ ID Nos. 201 to 300 consecutive to the nucleotide sequence CATG.

19. A human macrophage-expressed gene group comprising 30 genes expressed more in human macrophages differentiated through granulocyte-macrophage colony stimulating factor from human monocytes than in human monocytes, wherein the cDNAs of the individual genes individually contain nucleotide sequences of SQ ID Nos. 301 to 330 consecutive to the nucleotide sequence CATG closest to the polyA region.

20. A human macrophage-expressed gene group according to claim 19, wherein the difference between the expression frequency of the gene encoding the cDNA containing the nucleotide sequence of SQ ID No. 301 and the expression frequency of the same gene in human monocytes is the largest and the decreasing order of the expression frequencies of the remaining 29 genes is the order of SQ ID Nos. 302 to 330.

21. A human macrophage-expressed gene cDNA group comprising the cDNAs or partial sequences thereof of the individual genes composing a human macrophage-expressed gene group according to claim 19, wherein the cDNAs individually contain nucleotide sequences of SQ ID Nos. 301 to 330 consecutive to the nucleotide sequence CATG closest to the polyA region.

22. A human macrophage-expressed novel gene group included in a human macrophage-expressed gene group according to claim 19, wherein the cDNAs of the individual novel genes individually contain nucleotide sequences of SQ ID Nos. 309, 310, 312, 317, 318, 325, 327, 328 and 329.

23. Proteins encoded by the individual genes of a human macrophage-expressed novel gene group according to claim 22, and antibodies against the proteins.

24. A group of oligonucleotides individually consisting of nucleotide sequences of SQ ID Nos. 301 to 330 consecutive to the nucleotide sequence CATG.

25. A human monocyte-expressed gene group comprising 30 genes expressed more in human monocytes than in human macrophages differentiated through granulocyte-macrophage colony stimulating factor from human monocytes, wherein the cDNAs of the individual genes individually contain nucleotide sequences of SQ ID Nos. 331 to 360 consecutive to the nucleotide sequence CATG closest to the polyA region.

26. A human monocyte-expressed gene group according to claim 25, wherein the difference between the expression frequency of the gene encoding the cDNA containing the nucleotide sequence of SQ ID No. 331 and the expression frequency of the same gene in human macrophages is the largest and the decreasing order of the expression frequencies of the remaining 29 genes is the order of SQ ID Nos. 332 to 360.

27. A human monocyte-expressed gene cDNA group comprising the cDNAs or partial sequences thereof of the individual genes composing a human monocyte-expressed gene group according to claim 25, wherein the cDNAs individually contain nucleotide sequences of SQ ID Nos. 331 to 360 consecutive to the nucleotide sequence CATG closest to the polyA region.

28. A human monocyte-expressed novel gene group included in a human monocyte-expressed gene group according to claim 25, wherein the cDNAs of the individual novel genes individually contain nucleotide sequences of SQ ID Nos. 336, 339, 343 to 345, 347, 348, 351 to 353, 356 and 358 to 360.

29. Proteins encoded by the individual genes of a human monocyte-expressed novel gene group according to claim 28, and antibodies against the proteins.

30. A group of oligonucleotides individually consisting of nucleotide sequences of SQ ID Nos. 331 to 360 consecutive to the nucleotide sequence CATG.

31. A human macrophage-expressed gene group comprising 30 genes expressed more in human macrophages differentiated through macrophage colony stimulating factor from human monocytes than in human monocytes, wherein the cDNAs of the individual genes individually contain nucleotide sequences of SQ ID Nos. 361 to 390 consecutive to the nucleotide sequence CATG closest to the polyA region.

32. A human macrophage-expressed gene group according to claim 31, wherein the difference between the expression frequency of the gene encoding the cDNA containing the nucleotide sequence of SQ ID No. 361 and the expression frequency of the same gene in human monocytes is the largest and the decreasing order of the expression frequencies of the remaining 29 genes is the order of SQ ID Nos. 362 to 390.

33. A human macrophage-expressed gene cDNA group comprising the cDNAs or partial sequences thereof of the individual genes composing a human macrophage-expressed gene group according to claim 31, wherein the cDNAs individually contain nucleotide sequences of SQ ID Nos. 361 to 390 consecutive to the nucleotide sequence CATG closest to the polyA region.

34. A human macrophage-expressed novel gene group included in a human macrophage-expressed gene group according to claim 31, wherein the cDNAs of the individual novel genes individually contain nucleotide sequences of SQ ID Nos. 365, 366, 369, 373, 374, 377, 379 to 381, 384, 385 and 387.

35. Proteins encoded by the individual genes of a human macrophage-expressed novel gene group according to claim 34, and antibodies against the proteins.

36. A group of oligonucleotides individually consisting of nucleotide sequences of SQ ID Nos. 361 to 390 consecutive to the nucleotide sequence CATG.

37. A human monocyte-expressed gene group comprising 30 genes expressed more in human monocytes than in human macrophages differentiated through macrophage colony stimulating factor from human monocytes, wherein the cDNAs of the individual genes individually contain nucleotide sequences of SQ ID Nos. 391 to 420 consecutive to the nucleotide sequence CATG closest to the polyA region.

38. A human monocyte-expressed gene group according to claim 37, wherein the difference between the expression frequency of the gene encoding the cDNA containing the nucleotide sequence of SQ ID No. 391 and the expression frequency of the same gene in human macrophages is the largest and the decreasing order of the expression frequencies of the remaining 29 genes is the order of SQ ID Nos. 392 to 420.

39. A human monocyte-expressed gene cDNA group comprising the cDNAs or partial sequences thereof of the individual genes composing a human monocyte-expressed gene group according to claim 37, wherein the cDNAs individually contain nucleotide sequences of SQ ID Nos. 391 to 420 consecutive to the nucleotide sequence CATG closest to the polyA region.

40. A human monocyte-expressed novel gene group included in a human monocyte-expressed gene group according to claim 37, wherein the cDNAs of the individual novel genes individually contain nucleotide sequences of SQ ID Nos. 392, 393, 396, 398, 400, 401, 407, 411, 412, 415 and 417 to 419.

41. Proteins encoded by the individual genes of a human monocyte-expressed novel gene group according to claim 40, and antibodies against the proteins.

42. A group of oligonucleotides individually consisting of nucleotide sequences of SQ ID Nos. 391 to 420 consecutive to the nucleotide sequence CATG.

43. A human macrophage-expressed gene group comprising 30 genes expressed more in human macrophages differentiated through granulocyte-macrophage colony stimulating factor from human monocytes than in human macrophages differentiated through macrophage colony stimulating factor from human monocytes, wherein the cDNAs of the individual genes individually contain nucleotide sequences of SQ ID Nos. 421 to 450 consecutive to the nucleotide sequence CATG closest to the polyA region.

44. A human macrophage-expressed gene group according to claim 43, wherein the difference between the expression frequency of the gene encoding the cDNA containing the nucleotide sequence of SQ ID No. 421 and the expression frequency of the same gene in human macrophages differentiated through macrophage colony stimulating factor from human monocytes is the largest and the decreasing order of the expression frequencies of the remaining 29 genes is the order of SQ ID Nos. 422 to 450.

45. A human macrophage-expressed gene cDNA group comprising the cDNAs or partial sequences thereof of the individual genes composing a human macrophage-expressed gene group according to claim 43, wherein the cDNAs individually contain nucleotide sequences of SQ ID Nos. 421 to 450 consecutive to the nucleotide sequence CATG closest to the polyA region.

46. A human macrophage-expressed novel gene group included in a human macrophage-expressed gene group according to claim 43, wherein the cDNAs of the individual genes individually contain nucleotide sequences of SQ ID Nos. 421, 422, 426 to 433, 438 to 440, 443 to 446 and 448 to 450.

47. Proteins encoded by the individual genes of a human macrophage-expressed novel gene group according to claim 46, and antibodies against the proteins.

48. A group of oligonucleotides individually consisting of nucleotide sequences of SQ ID Nos. 421 to 450 consecutive to the nucleotide sequence CATG.

49. A human macrophage-expressed gene group comprising 30 genes expressed more in human macrophages differentiated through macrophage colony stimulating factor from human monocytes than in human macrophages differentiated through granulocyte-macrophage colony stimulating factor from human monocytes, wherein the cDNAs of the individual genes individually contain nucleotide sequences of SQ ID Nos. 451 to 480 consecutive to the nucleotide sequence CATG closest to the polyA region.

50. A human macrophage-expressed gene group according to claim 49, wherein the difference between the expression frequency of the gene encoding the cDNA containing the nucleotide sequence of SQ ID No. 451 and the expression frequency of the gene in human macrophages differentiated through granulocyte-macrophage colony stimulating factor from human monocytes is the largest and the decreasing order of the expression frequencies of the remaining 29 genes is the order of SQ ID Nos. 452 to 480.

51. A human macrophage-expressed gene cDNA group comprising the cDNAs or partial sequences thereof of the individual genes composing a human macrophage-expressed gene group according to claim 49, wherein the cDNAs individually contain nucleotide sequences of SQ ID Nos. 451 to 480 consecutive to the nucleotide sequence CATG closest to the polyA region.

52. A human macrophage-expressed novel gene group included in a human macrophage-expressed gene group according to claim 49, wherein the cDNAs of the individual novel genes individually contain nucleotide sequences of SQ ID Nos. 451, 453, 454, 456 to 458, 461 to 464, 466 to 469, 471 to 473, 475 and 478 to 480.

53. Proteins encoded by the individual genes of a human macrophage-expressed novel gene group according to claim 52, and antibodies against the proteins.

54. A group of oligonucleotides individually consisting of nucleotide sequences of SQ ID Nos. 451 to 480 consecutive to the nucleotide sequence CATG.
